# EUROPEAN PATENT SPECIFICATION

(11) **EP 3 083 975 B1**
(45) Date of publication and mention of the grant of the patent: **14.11.2018**
(21) Application number: 14816252.2
(22) Date of filing: 19.12.2014
(51) Int. Cl.: C12P 17/02, C07D 311/00, C12N 9/16, C12N 15/52

(54) **STEREO-SPECIFIC SYNTHESIS OF (13R)-MANOYL OXIDE**
STEREOSPEZIFISCHE SYNTHESE VON (13R)-MANOYLOXID
SYNTHÈSE STÉRÉO-SPÉCIFIQUE DE (13R)-MANOYL OXYDE

(30) Priority: 20.12.2013 EP 13198742; 20.12.2013 EP 13198756
(43) Date of publication of application: 26.10.2016
(73) Proprietor: Technical University of Denmark, 2800 Kgs. Lyngby (DK); University of Copenhagen, 1165 Copenhagen K (DK)
(72) Inventor: HAMBERGER, Björn, 48864 Okemos Michigan (US); PATERAKI, Eirini, DK-2450 Copenhagen SV (DK); MØLLER, Birger Lindberg, DK-2700 Brønshøj (DK); NØRHOLM, Morten, DK-3400 Hillerød (DK); NIELSEN, Morten Thrane, DK-2200 Copenhagen N (DK); ANDERSEN-RANBERG, Johan, 94705 Berkeley California (US); BOHLMANN, Carl Jörg, Vancouver, British Columbia V6S 1T4 (CA); ZERBE, Philipp, North Vancouver, British Columbia V7G 1C3 (CA)
(74) Representative: Rees, Kerry
(86) International application number: PCT/EP2014/078728
(87) International publication number: WO 2015/091943

(56) References cited:
- WO-A-2009/044336
- WO-A1-2009/101126
- CN-A- 101 538 576
- GÜNNEWICH ET AL: "A diterpene synthase from the clary sage Salvia sclarea catalyzes the cyclization of geranylgeranyl diphosphate to (8R)-hydroxy-copalyl diphosphate", PHYTOCHEMISTRY, vol. 91, 6 September 2012 (2012-09-06), pages 93-99, XP002724040, cited in the application
- ZERBE ET AL: "Gene discovery of modular diterpene metabolism in nonmodel systems", PLANT PHYSIOLOGY, vol. 162, 23 April 2013 (2013-04-23), pages 1073-1091, XP002724041, cited in the application
- CANIARD ET AL: "Discovery and functional characterization of two diterpene synthases for sclareol biosynthesis in Salvia sclarea (L.) and their relevance for perfume manufacture", BMC PLANT BIOLOGY, vol. 12, 26 July 2012 (2012-07-26), pages 119(1)-131(13), XP021128059, cited in the application
- SCHALK ET AL: "Toward a biosynthetic route to sclareol and amber odorants", JOURNAL OF THE AMERICAN CHEMICAL SOCIETY, vol. 134, 2012, pages 18900-18903, XP002724077,
- ASADA ET AL: "Labdane-type diterpenoids from hairy root cultures of Coleus forskohlii, possible intermediates in the biosynthesis of forskolin", PHYTOCHEMISTRY, vol. 79, 2012, pages 141-146, XP028428592,
- AZUMA ET AL: "Floral scent emissions from Asarum yaeyamense and related species", BIOCHEMICAL SYSTEMATICS AND ECOLOGY, vol. 38, 2010, pages 548-553, XP027437736,
- PATERAKI ET AL: "Manoyl oxide (13R), the biosynthetic precursor of forskolin, is synthesized in specialized root cork cells in Coleus forskohlii", PLANT PHYSIOLOGY, vol. 164, March 2014 (2014-03), pages 1222-1236, XP002724033,
- NIELSEN ET AL: "Microbial synthesis of the forskolin precursor manoyl oxide in enantiomerically pure form", APPLIED ENVIRONMENTAL MICROBIOLOGY (AEM ACCEPTS), 19 September 2014 (2014-09-19), pages 1-26, XP002735928,
- DEMETZOS ET AL: "A simple and rapid method for the differentiation of C-13 manoyl oxide epimers in biologically important samples using GC-MS analysis supported with NMR spectroscopy and computational chemistry results", BIOORGANIC & MEDICINAL CHEMISTRY LETTERS, vol. 12, 2002, pages 3605-3609, XP002724824, cited in the application
- CUI ET AL: "Candidate genes involved in tanshinone biosynthesis in hairy roots of Salvia miltiorrhiza revealed by cDNA microarray", MOLECULAR BIOLOGY REPORTS, vol. 38, 2011, pages 2471-2478, XP019894184,
- CAMBIE ET AL: "Conversion of manoyl oxide into 12-beta-hydroxymanoyl oxide", AUSTRALIAN JOURNAL OF CHEMISTRY, vol. 44, 1991, pages 469-475,
- MEDINI ET AL: "Antibacterial activity and phytochemical composition of leaf and berry essential oils of two Juniperus phoenicea subspecies gathered in Tunisia", JOURNAL OF EXPERIMENTAL BIOLOGY AND AGRICULTURAL SCIENCES, vol. 1, July 2013 (2013-07), pages 166-173,

## Description

### Field of invention

The present invention relates to a method for manufacturing enantiomerically pure (13R)-manoyl oxide, said method comprising the steps of contacting geranylgeranyl diphosphate (GGPP) with a class II diterpene synthase to obtain labd-13-en-8,15-diol diphosphate (LPP), and then contacting the LPP with a class I diterpene synthase to obtain (13R)-manoyl oxide. The invention further relates to (13R)-manoyl oxide obtained by the method of the invention, wherein the (13R)-manoyl oxide is more than 90% enantiomerically pure. The invention furthermore relates to polypeptides with diterpene synthase activity. The invention further relates to polynucleotides encoding such polypeptides. Also provided are vectors for expression of the polypeptides and host cells expressing the polypeptides. Such polypeptides may be useful in aforementioned methods.

### Background of invention

Manoyl oxide is a compound which exhibits a number of important properties like antibacterial, anticancer and anti-inflammatory activities. Manoyl oxide has so far only been detected as a side product or artefact of some reactions, and was present in a racemic mixture. Manoyl oxide derivatives also present numerous attractive properties. The (13R)-manoyl oxide epimer is a putative precursor of forskolin, a labdane diterpenoid found in the root of *Coleus forskohlii* (family *Lamiaceae*) which has received much attention for its broad range of pharmacological activities. However, despite extensive studies focusing on its medicinal properties, the biosynthesis of forskolin has not yet been conclusively elucidated.
*Coleus forskohlii* (synonym: *Plectranthus barbatus*) is a perennial medicinal shrub of the mint family (Lamiaceae) indigenous to the subtropical and temperate climate zones of India and south-east Asia. The plant has been used since ancient times in Hindu and Ayurvedic traditional medicine for treating a broad range of human health disorders.
The main active compound of *C. forskohlii* is forskolin, a heterocyclic labdane type diterpene found in the roots of the plant. The diverse pharmaceutical known and potential applications for forskolin extend from alleviation of glaucoma, anti-HIV or antitumor activities to treatment of hypertension and heart failure. The efficacy of forskolin relies on activation of the adenylate cyclase enzyme leading to a marked increase of the intracellular level of cAMP (3'-5'-cyclic adenosine monophosphate) in mammalian in vitro and in vivo systems. The semi-synthetic forskolin derivative NKH477 has been approved for commercial use in Japan for treatment of cardiac surgery complications, heart failure, and cerebral vasospasm, while a forskolin eye drop solution was recently approved as an effective treatment for glaucoma.

The chemical complexity of C. forskohlii has been well studied and a total of 68 different diterpenoids have been isolated and identified from different tissues of the plant, of which 25 belong to the class of abietanes and 43 to the class of labdanes. While the tricyclic abietane diterpenes have been reported to accumulate predominantly in the aerial parts, labdane diterpenoids with a bicyclic decalin core were detected primarily in the roots. Forskolin is a representative of an unusual series of tricyclic (8,13)-epoxy-labdanes, characteristic for this plant.

Manoyl-oxide has to this date only been detected as experimental artefact (Zerbe et al., 2012; Günnewich et al., 2013). Enzymatic conversion leading to production of manoyl oxide has at present never been reported. Thus pure enantiomers of manoyl oxide are currently not available. Methods of producing enantiomerically pure enantiomers of manoyl-oxide, including (13R)-manoyl oxide are needed. Also polypeptides capable of producing manoyl oxide are needed.

GÜNNEWICH ET AL: "A diterpene synthase from the clary sage Salvia sclarea catalyzes the cyclization of geranylgeranyl diphosphate to (8R)-hydroxy-copalyl diphosphate", PHYTOCHEMISTRY, vol. 91, 6 September 2012 (2012-09-06), pages 93 - 99, discloses that the diterpene synthase SscTPS1 from glandular trichomes of Clary sage catalyzes cyclization of geranylgeranyl diphosphate by a mechanism that introduces oxygen to form (8R)-hydroxy-copalyl diphosphate, which can then be converted to sclareol and 13-episclareol by acid hydrolysis.

ZERBE ET AL: "Gene discovery of modular diterpene metabolism in nonmodel systems", PLANT PHYSIOLOGY, vol. 162, 23 April 2013 (2013-04-23), pages 1073 - 1091, discloses that specialized diterpene pathways are extremely diverse across the plant kingdom, and most specialized diterpenes are taxonomically restricted to a few plant species, genera, or families. This document also discloses an approach using a set of 10 different plant species to develop a general strategy for diterpene gene discovery in nonmodel systems. The approach combines metabolite-guided transcriptome resources, custom diterpene synthase (diTPS) and cytochrome P450 reference gene databases, phylogenies, and, single and coupled enzyme assays using microbial and plant expression systems. In the 10 species, 46 new diTPS candidates were discovered along with over 400 putatively terpenoid-related P450s in a resource of nearly 1 million predicted transcripts of diterpene-accumulating tissues. For example, GrTPS1 and GrTPS6 are disclosed. These Gr enzymes are very different from the TPS enzymes disclosed herein. Thus, GrTPS1 only shares 43% sequence identity with CfTPS2, whereas GrTPS6 shares as little as 34% and 32% sequence identity with CfTPS3 and CfTPS4, respectively.

CANIARD ET AL: "Discovery and functional characterization of two diterpene synthases for sclareol biosynthesis in Salvia sclarea (L.) and their relevance for perfume manufacture", BMC PLANT BIOLOGY, vol. 12, 26 July 2012 (2012-07-26), pages 119(1) - 131(13), discloses that SsLPPS and SsSS are two monofunctional diTPSs which, together, produce the diterpenoid specialized metabolite sclareol in a two-step process. They represent two of the first characterized hydroxylating diTPSs in angiosperms and generate the dihydroxylated labdane sclareol without requirement for additional enzymatic oxidation by activities such as cytochrome P450 monoxygenases. Yeast-based production of sclareol by co-expresssion of SsLPPS and SsSS was efficient enough to warrant the development and use of such technology for the biotechnological production of scareol and other oxygenated diterpenes.

SCHALK ET AL: "Toward a biosynthetic route to sclareol and amber odorants", JOURNAL OF THE AMERICAN CHEMICAL SOCIETY, vol. 134, 2012, pages 18900 - 18903, discloses the cloning and functional characterization of the enzymes responsible for the biosynthesis of sclareol. Furthermore, this document discloses the reconstruction of the sclareol biosynthetic pathway in genetically engineered Escherichia coli and reached sclareol titers of ∼ 1.5 g/L in high-cell-density fermentation, as a potential a basis for the development of an alternative, sustainable, and cost-efficient route to sclareol and other diterpene analogues.

PATERAKI ET AL: "Manoyl oxide (13R), the biosynthetic precursor of forskolin, is synthesised in specialised present Cork cells in Coleis forskohlii", PLANT PHYSIOLOGY, vol, 164, March 2014, pages 1222-1236 discloses the detection of forskolin in the root cork of C. forskohlii in a specialized cell type containing characteristic structures with histochemical properties consistent with oil bodies. Organelle purification and chemical analysis confirmed the localization of forskolin and of its simplest diterpene precursor backbone, (13R) manoyl oxide, to the oil bodies. Also disclosed is the functional characterization of four CfTPSs using in vitro and in planta assays. CfTPS2, which synthesizes the intermediate copal-8-ol diphosphate, in combination with CfTPS3 resulted in the stereospecific formation of (13R) manoyl oxide, while the combination of CfTPS1 and CfTPS3 or CfTPS4 led to formation of miltiradiene, precursor of abietane diterpenoids in C. forskohlii.

NIELSEN ET AL: "Microbial synthesis of the forskolin precursor manoyl oxide in enantiomerically pure form", APPLIED ENVIRONMENTAL MICROBIOLOGY (AEM ACCEPTS), 19 September 2014, pages 1-26 discloses a microbial synthesis of (13R)-manoyl oxide, a proposed intermediate in the biosynthesis of forskolin and other medically important labdane-type terpenoids. CUI ET AL: "Candidate genes involved in tanshinone biosynthesis in hairy roots of Salvia miltiorrhiza revealed by cDNA microarray", MOLECULAR BIOLOGY REPORTS, 2011, pages 2471-2478, discloses sequences about 75% identical with SEQ ID NOS 3-4.

### Summary of invention

In one aspect, the invention relates to a method of manufacturing (13R)-manoyl oxide, said method comprising the steps of:
(i) providing geranylgeranyl diphosphate (GGPP);
(ii) contacting GGPP of step (i) with a first polypeptide having a sequence at least 75% identical to SEQ ID NO: 1 [CfTPS2] or SEQ ID NO: 2 [SsLPPS], thus obtaining labd-13-en-8,15-diol diphosphate (LPP);
(iii) contacting the LPP of step (ii) with a second polypeptide having a sequence at least 75% identical to SEQ ID NO: 3 [CfTPS4], SEQ ID NO: 4 [CfTPS3] or SEQ ID NO: 5 [EpTPS8]; thus obtaining (13R)-manoyl-oxide.

In another aspect, the invention relates to (13R)-manoyl oxide obtained by the method of the invention, wherein the (13R)-manoyl oxide is more than 90% enantiomerically pure.

In another aspect, the invention relates to polypeptides having a diterpene synthase (diTPS) activity, as defined in claim 14. The invention further relates to polynucleotides encoding such polypeptides and to vectors comprising such polynucleotides, as defined in claims 19 and 21. The invention finally relates to a host cell comprising such vectors and/or such polynucleotides, as defined in claim 24. The polypeptides of the invention are relevant for catalysing enzymatic synthesis of manoyl oxide.

Thus, in one aspect, the invention relates to polypeptides having a diterpene synthase activity and comprising:
i) an amino acid sequence selected from the group consisting of SEQ ID NO: 1 [CfTPS2], SEQ ID NO: 4 [CfTPS3], SEQ ID NO: 3 [CfTPS4] and SEQ ID NO: 5 [EpTPS8];
ii) a biologically active sequence variant of said polypeptide, wherein the sequence variant has at least 90% sequence identity to said SEQ ID NO: 1 [CfTPS2], SEQ ID NO: 4 [CfTPS3], SEQ ID NO: 3 [CfTPS4] or SEQ ID NO: 5 [EpTPS8].

In a further aspect, the invention relates to a polynucleotide encoding a polypeptide of the invention.

In another aspect, the invention relates to a vector comprising at least one polynucleotide of the invention.

In yet another aspect, the invention relates to a host cell comprising a polynucleotide of the invention and/or a vector of the invention.

### Description of Drawings

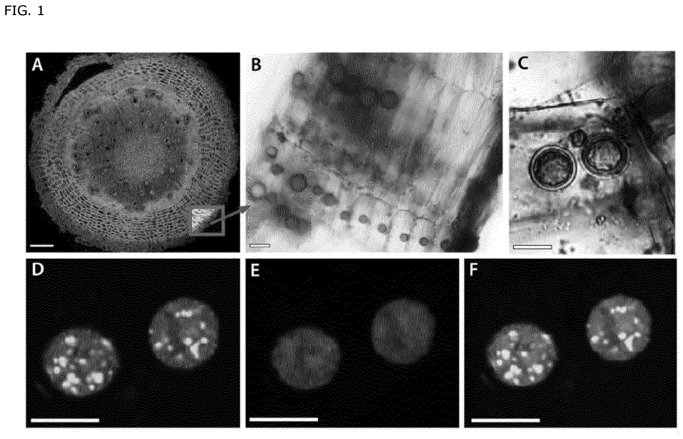

**Figure 1****:** Localization of oil bodies within the root cork of *C. forskohlii.* (A) Cross section of entire root with thick fissured cork. Lower right inset: the location of cork cells. (B) Rows of cork cells of different intensity, each with one prominent oil body. (C-F) Confocal imaging of Nile Red labeled oil bodies. (C) Cell with two oil bodies (same motif as D-F) seen in transmission channel. Discrimination between neutral lipids seen as bright deposits (D) and polar lipids seen in the magenta spectrum (E), also shown as overlay (F). The bar represents 200 µm (A) and 10 µm (B-F).

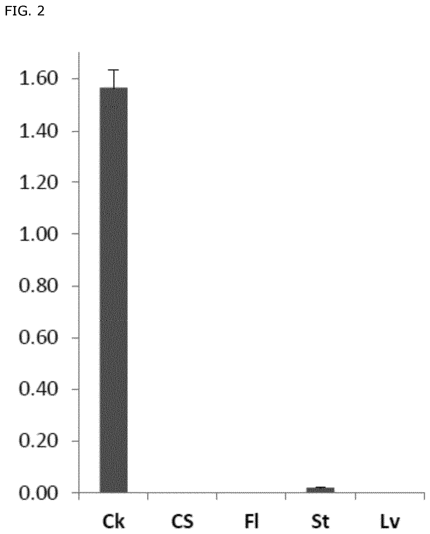

**Figure 2****:** Forskolin content (mg g⁻¹DW) as determined by HPLC-ELSD analysis from different tissues of *C. forskohlii.* (Ck), root cork; (CS), root stele and cortex; (FI), flowers; (St) stems and (Lv) leaves. Data are the mean ± SE of three independent biological replicates. The Y axis shows the forskolin content (mg/g dry weight).

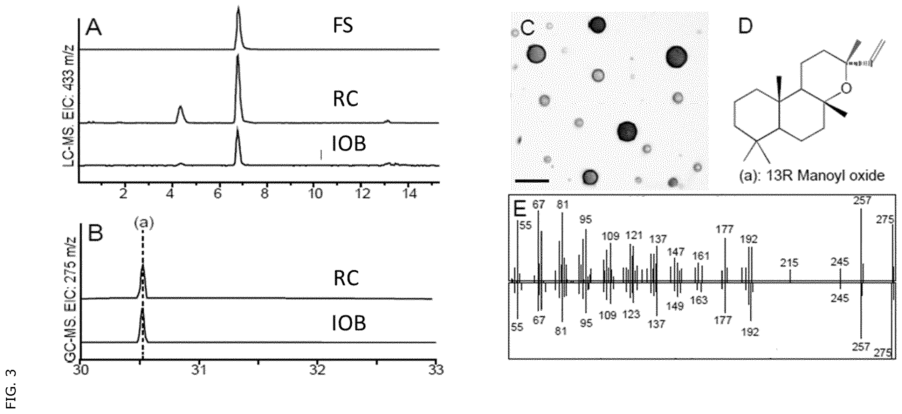

**Figure 3****:** Selected diterpenes detected in *C. forskohlii* oil bodies. FS: forskolin standard; RC: root cork; IOL: isolated oil bodies. (A) LC-MS analysis of forskolin (410 [forskolin]+23 [Na+]) in isolated oil bodies and in root cork tissue from *C. forskohlii.* The X axis shows the time in minutes. (B) GC-MS analysis of manoyl oxide in isolated oil bodies and in root cork tissue from *C. forskohlii.* The X axis shows the time in minutes. (C) Bright field microscope image of isolated *C. forskohlii* oil bodies. The bar represents 5 µm. (D) Molecular structure of (13R)-manoyl oxide. (E) Mass spectrum obtained from manoyl oxide identified in root cork tissue (top) and reference spectrum (bottom) from Wiley mass spectrum database.

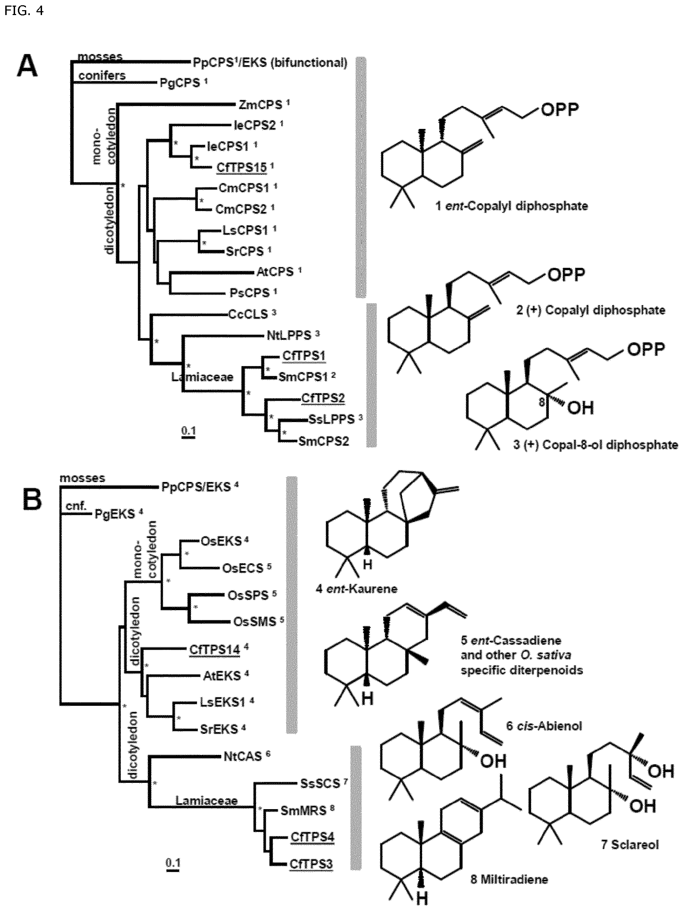

**Figure 4****:** Phylogenetic classification of *C. forskohlii* diterpene synthases with known class II (A) and class I (B) sequences. The phylograms are rooted with the bifunctional *ent*-copalyl diphosphate synthase/*ent*-kaurene synthase from the moss *Physcomitrella patens.* Asterisks indicate nodes supported by >80% bootstrap confidence and the scale bar indicates 0.1 amino acid changes. The numbers indicated at each enzyme are referred to the enzymatic products, the structures of which are given on the right.

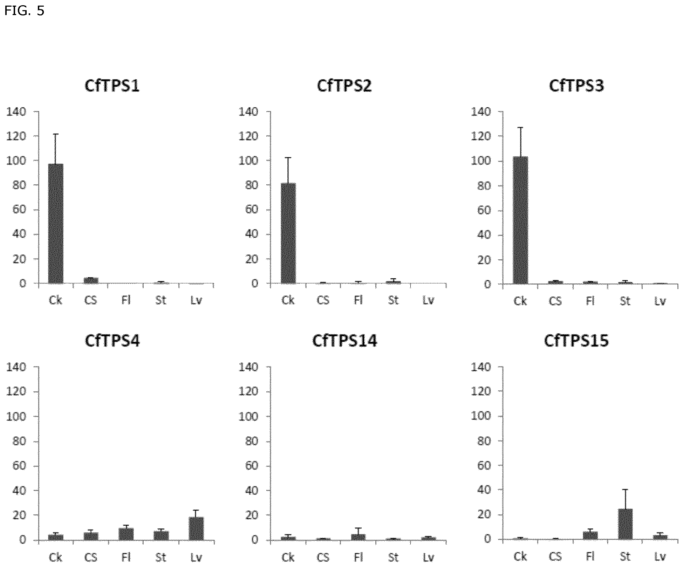

**Figure 5****:** Relative expression of *CfTPS* genes in *C. forskohlii* tissues. (Ck), root cork; (CS), root stele and cortex; (FI), flowers; (St), stems and (Lv), leaves. Transcript abundance of *CfTPS* genes expressed in arbitrary units was measured by qPCR using the translation initiation factor (TIF4a) for normalization. Each value represents the average of three biological replicates, each of which was performed in at least three technical replicates.

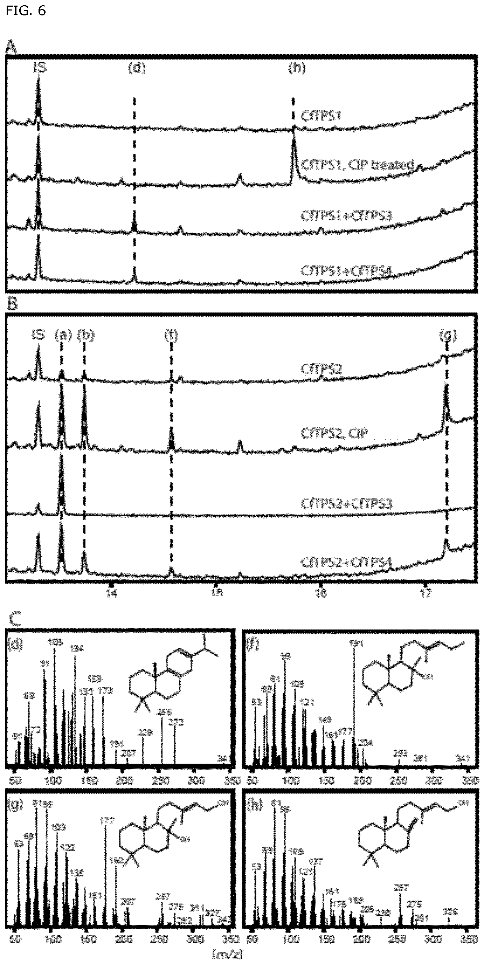

**Figure 6****:** GC-MS analysis of *in vitro* assays with *C. forskohlii* diTPS. IS, internal standard (1ppm 1-eicosene). (A) *In vitro* assays with CfTPS2 alone and coupled assays with CfTPS2 and CfTPS3 and CfTPS4. Extracts of CfTPS2 assays were treated with calf intestinal alkaline phosphatase (CIP). The X axis shows the retention time (minutes). (B) *In vitro* assays with CfTPS1 and coupled with CfTPS3 and CfTPS4. Extracts of CfTPS1 were treated with CIP. The X axis shows the retention time (minutes). (a), (13R)-manoyl oxide; (b), (13S)-manoyl oxide; (g), labd-13-en-8,15-diol and (f), labden-8-ol; (d), miltiradiene and (h), copal-15-ol. (C) Mass spectra of compounds identified from assays. Structures tentatively identified as described in Materials and Methods.

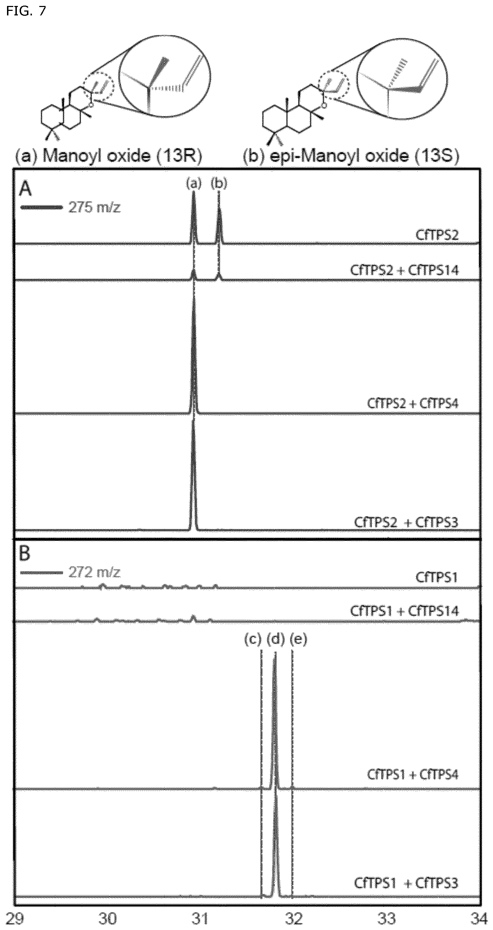

**Figure 7****:** GC-MS analysis of hexane extracts from *N. benthamiana* transiently expressing *C. forskohlii* diTPS. (A) Extracted ion chromatogram of EIC: 275 m/z. (a), (13R)-manoyl oxide and (b), (13S)-manoyl oxide. (B) Extracted ion chromatogram of EIC: 272 m/z. (c), dehydroabietadiene; (d), miltiradiene and trace amount of (e), abietadiene.

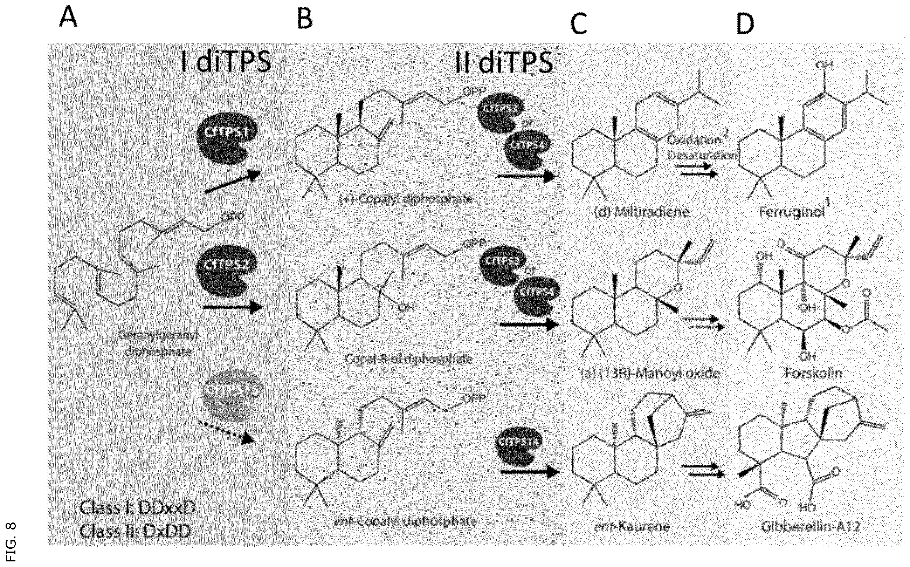

**Figure 8****:** Scheme of the biosynthetic routes from GGPP to specialized and general diterpenoids of the abietane, labdane and *ent-kaurene* class. Dashed arrows indicate reactions without experimental evidence in *C. forskohlii.* ¹Detection of (+)-ferruginol in *C. forskohlii* was reported earlier (Kelecom, 1983); ²CYP76AH1 from the close relative *Salvia miltiorrhiza* was shown to convert miltiradiene to ferruginol (Guo et al., 2013). A: universal precursor; B: diphosphate intermediates; C: diterpene backbone; D: representative functional diterpenoids.

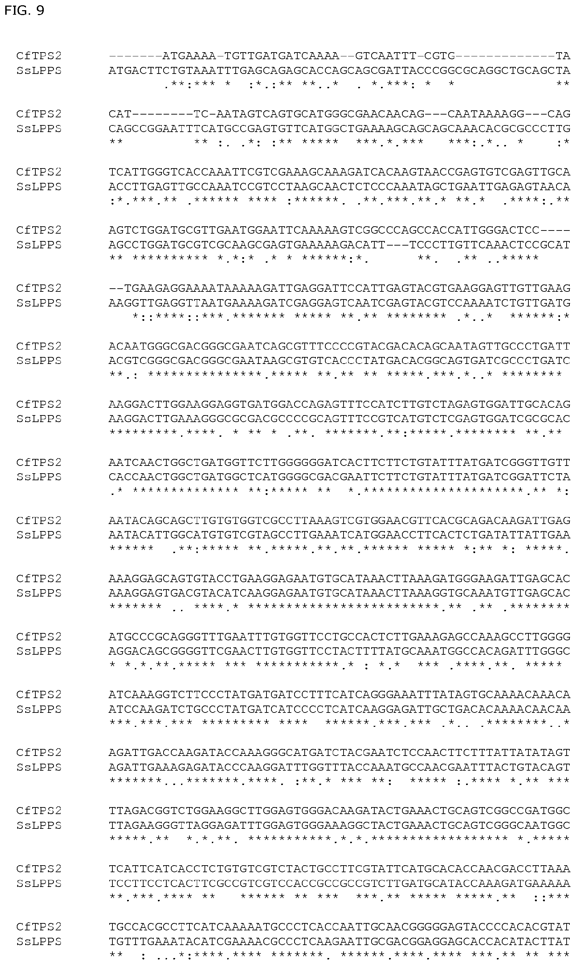

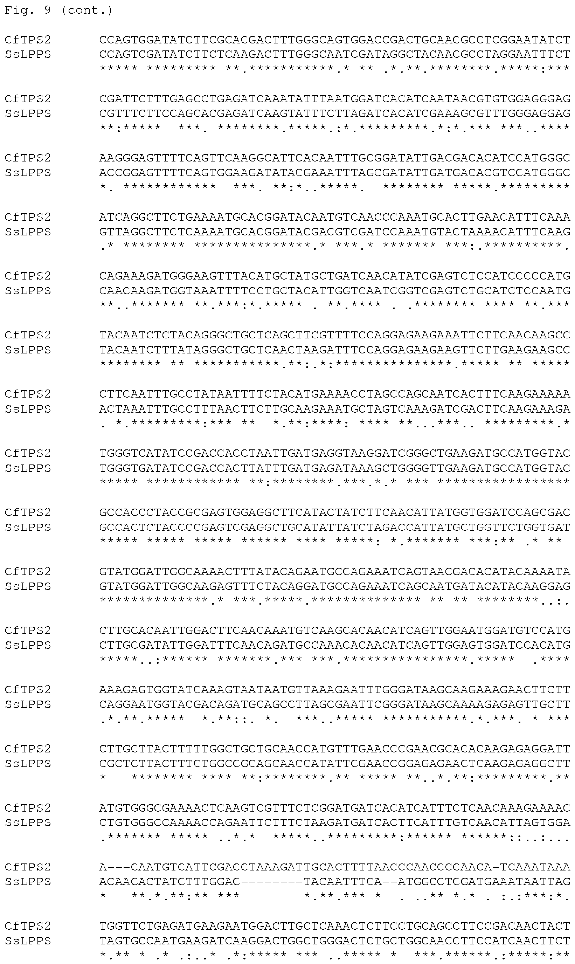

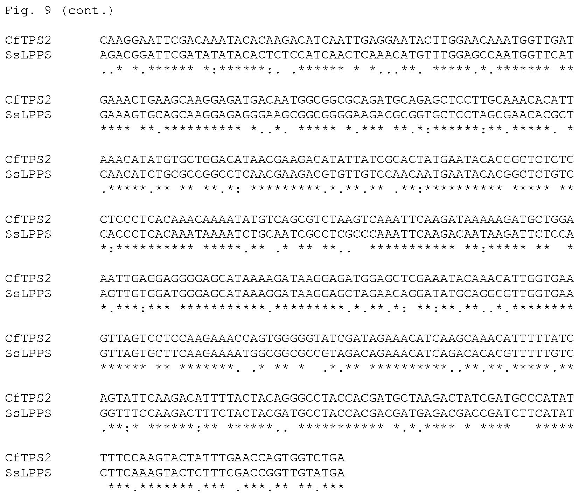

**Figure 9****:** Clustal alignment of the class II diTPS. The cDNA sequences encoding CfTPS2 and SsLPPS were aligned using the Clustal omega from the EMBL-EBI (http://www.ebi.ac.uk/Tools/msa/clustalo/help/).

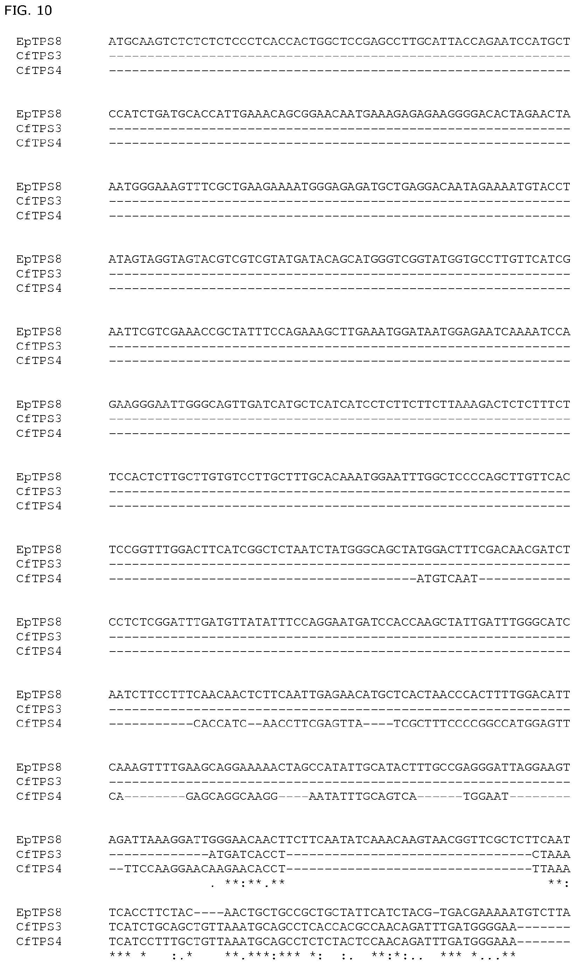

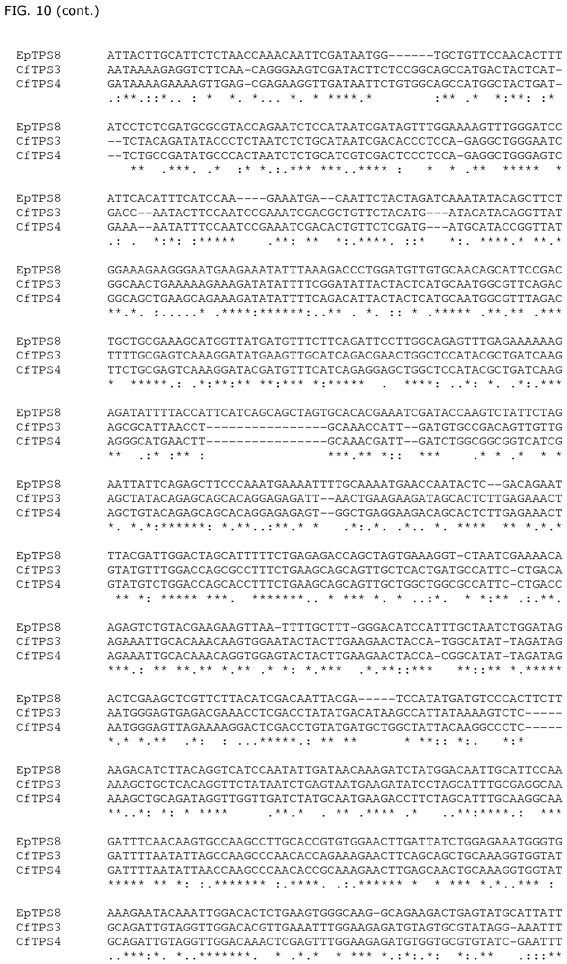

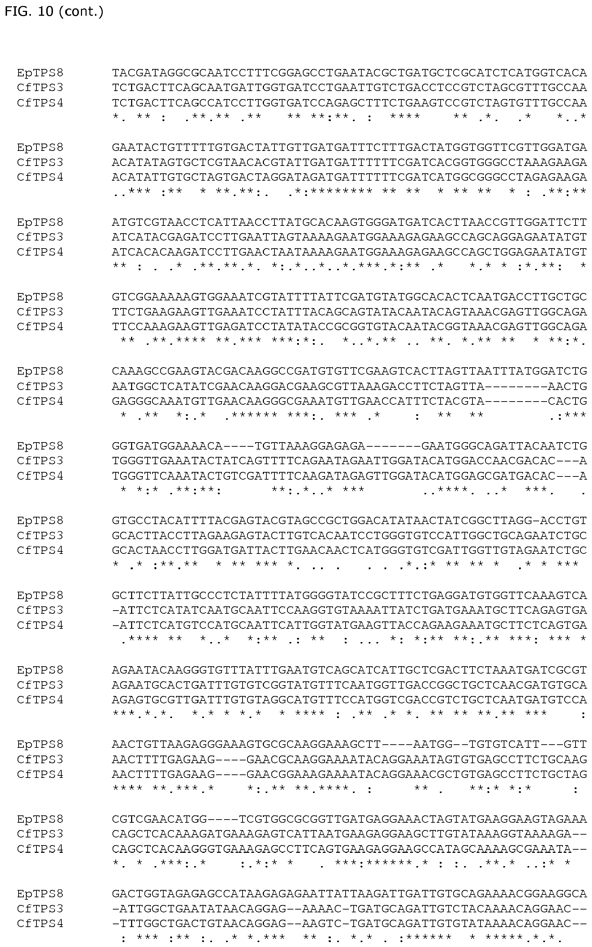

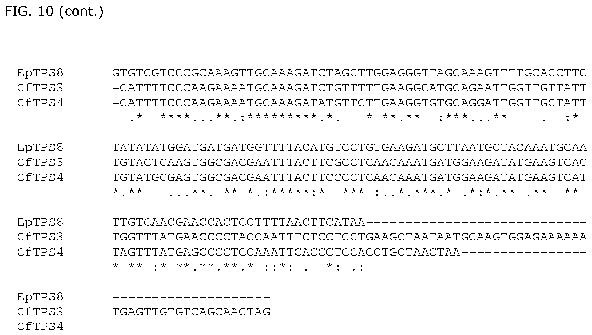

**Figure 10****:** Clustal alignment of the class I diTPS. The cDNA sequences encoding CfTPS3, CfTPS4 and EpTPS8 were aligned using the Clustal omega from the EMBL-EBI (http://www.ebi.ac.uk/Tools/msa/clustalo/help/).

### Detailed description of the invention

In one aspect, the invention relates to a method of manufacturing (13R)-manoyl oxide, said method comprising the steps of:
(i) providing geranylgeranyl diphosphate (GGPP);
(ii) contacting GGPP of step (i) with a first polypeptide having the sequence of SEQ ID NO:1 [CfTPS2], or SEQ ID NO: 2 [SsLPPS], thus obtaining labd-13-en-8,15-diol diphosphate (LPP);
(iii) contacting the LPP of step (ii) with a second polypeptide having the sequence of SEQ ID NO: 3 [CfTPS4], SEQ ID NO: 4 [CfTPS3] or SEQ ID NO: 5 [EpTPS8] or a biologically active sequence variant of said polypeptide, wherein the sequence variant has at least 75% sequence identity to SEQ ID NO: 3 [CfTPS4], SEQ ID NO: 4 [CfTPS3] or SEQ ID NO: 5 [EpTPS8];
thus obtaining (13R)-manoyl-oxide.

In another aspect, the invention relates to (13R)-manoyl oxide obtained by the method of the invention, wherein the (13R)-manoyl oxide is more than 90% enantiometrically pure.

In one aspect the invention relates to polypeptides having a diterpene synthase (diTPS) activity, as defined in claim 14. The invention further relates to polynucleotides encoding such polypeptides and to vectors comprising such polypeptides, as defined in claims 19 and 23. The invention finally relates to a host cell comprising such vectors and/or such polynucleotides, as defined in claim 24. The polypeptides of the invention are relevant for catalysing enzymatic synthesis of manoyl oxide.

### Definitions

Amino acid: Any synthetic or naturally occurring amino carboxylic acid, including any amino acid occurring in peptides and polypeptides including proteins and enzymes synthesized in vivo thus including modifications of the amino acids. The term amino acid is herein used synonymously with the term "amino acid residue" which is meant to encompass amino acids as stated which have been reacted with at least one other species, such as 2, for example 3, such as more than 3 other species. The generic term amino acid comprises both natural and non-natural amino acids any of which may be in the "D" or "L" isomeric form.
Diterpene synthases (diTPS): Diterpene synthases (diTPS, EC 4.2.3.X) are enzymes capable of synthesising diterpene olefins and alcohols by sequential cycloisomerisation of the substrate geranylgeranyl-diphosphate (GGPP). DiTPS can be sorted in two classes, depending on the presence of a conserved motif. Class I diTPS contain an active site with a DDxxD motif, where D is an aspartic acid and x is any amino acid. Class II diTPS contain an active site with a DxDD motif, where D is an aspartic acid and x is any amino acid. Bifunctional classI/II diTPS contain two active sites, with a DDxxD and a DxDD motif, respectively.
Diterpenoid: As used herein, a diterpenoid is an unsaturated hydrocarbon based on the isoprene unit (C₅H₈), and having a general formula C_{5X}H_{8X}. A diterpene contains a backbone of 20 carbon atoms, which can be decorated by additional groups, e.g. by esterification. A diterpenoid also is a type of diterpene. A diterpenoid can derive from geranylgeranyl pyrophosphate (GGPP). Diterpenoids include all types of molecules derived from GGPP with a very broad range of functionalization. Examples of diterpenoids are olefins and diterpene alcohols.
Enantiomer: An enantiomer or enantiomorph or epimer is one of two stereoisomers that are mirror images of each other that are non-superposable. In other words, an enantiomer is a chiral molecule having a non-superposable mirror image. Enantiomers have, when present in a symmetric environment, identical chemical and physical properties except for their ability to rotate plane-polarized light (+/-) by equal amounts but in opposite directions. Enantiomers of one compound often react differently with other substances that are also enantiomers. Since many molecules in the living organisms are enantiomers themselves, there is sometimes a marked difference in the effects of two enantiomers on these organisms. In drugs, for example, often only one of a drug's enantiomers is responsible for the desired physiologic effects, while the other enantiomer is less active, inactive, or sometimes even responsible for adverse effects. Thus drugs composed of only one enantiomer (enantiomerically pure) can be developed to enhance the pharmacological efficacy and possibly dampen some side effects.
Enantiomerically pure: Enantiomerically pure, or enantiopure, refers to samples having, within the limits of detection, molecules of only one chirality.
Fragment: is used to indicate a non-full length part of a polynucleotide or polypeptide. Thus, a fragment is itself also a polynucleotide or polypeptide, respectively.
Identity: The determination of percent identity between sequences such as polynucleotide sequences or amino acid sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the BLASTN and BLASTP programs of Altschul, et al. (1990) J. Mol. Biol. 215:403-410. In order to characterize the identity, subject sequences are aligned so that the highest order homology (match) is obtained. Based on these general principles, the "percent identity" of two polynucleotide sequences may be determined using the BLASTN algorithm [Tatiana A. Tatusova, Thomas L. Madden: Blast 2 sequences - a new tool for comparing protein and nucleotide sequences; FEMS Microbiol. Lett. 1999 174 247-250], which is available from the National Center for Biotechnology Information (NCBI) web site (http://www.ncbi.nlm.nih.gov), and using the default settings suggested here (i.e. Reward for a match = 1; Penalty for a mismatch = -2; Strand option = both strands; Open gap = 5; Extension gap = 2; Penalties gap x_dropoff = 50; Expect = 10; Word size = 11; Filter on). The BLASTN algorithm determines the % sequence identity in a range of overlap between two aligned nucleotide sequences. Another preferred, non-limiting example of a mathematical algorithm utilized for the comparison of sequences is the CLUSTAL W (1.7) alignment algorithm (Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680.). CLUSTAL W can be used for multiple sequence alignment preferably using BLOSUM 62 as scoring matrix. When calculating sequence identities, CLUSTAL W includes any gaps made by the alignment in the length of the reference sequence. Sequence identities are calculated by dividing the number of matches by the length of the aligned sequences with gaps. In general, the sequence identity is calculated with reference to the entire length of the reference sequence. Thus, a candidate sequence sharing 80% amino acid identity with a reference sequence, requires that, following alignment, 80% of the amino acids in the candidate sequence are identical to the corresponding amino acids in the reference sequence.
Operative: An operative domain in relation to a class I or class II domain refers to a domain securing the biological processing of the polypeptide.
Plastidial targeting signal: A short sequence of amino acids that determines that a polypeptide should locate to the plastid in a plant cell.
Polynucleotide: A chain or sequence of nucleotides that convey genetic information. In regards to the present invention the polynucleotide is a deoxyribonucleic acid (DNA).
Polypeptide: Plurality of covalently linked amino acid residues defining a sequence and linked by amide bonds. The term is used analogously with oligopeptide and peptide. The natural and/or non-natural amino acids may be linked by peptide bonds or by non-peptide bonds. The term peptide also embraces post-translational modifications introduced by chemical or enzyme-catalyzed reactions, as are known in the art. The term can refer to a variant or fragment of a polypeptide.
Promoter: A binding site in a DNA chain at which RNA polymerase binds to initiate transcription of messenger RNA by one or more nearby structural genes. An inducible promoter refers to a promoter where initiation of transcription can be induced by e.g. addition of a compound to the growth medium or by changing the temperature.
Racemic mixture: A racemic mixture contains equal parts of an optically active isomer and its enantiomer and has zero net rotation of plane-polarized light.
Substantially pure: As used herein, substantially pure means sufficiently homogeneous to appear free of readily detectable impurities as determined by standard methods of analysis, such as thin layer chromatography (TLC), gel electrophoresis and high performance liquid chromatography (HPLC), gas-chromatography mass-spectrometry (GC-MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound can, however, be a mixture of stereoisomers or isomers. In such instances, further purification might increase the specific activity of the compound.
Transient expression: Transient expression refers to temporary expression of a polypeptide, for a limited period of time. Transient expression can be controlled by inducible and repressible promoters, agroinfiltration of plant cells with a bacterium such as *Agrobacterium tumefaciens,* and other methods known in the art.
Variant: a 'variant' of a given reference polynucleotide or polypeptide refers to a polynucleotide or polypeptide that displays a certain degree of sequence homology/identity to said reference polynucleotide or polypeptide but is not identical to said reference polynucleotide or polypeptide.
Vector: A vector is a DNA molecule or an organism comprising a DNA molecule used as a vehicle to artificially carry foreign genetic material into another cell, where the DNA molecule can be replicated and/or expressed. The vectors herein may be plasmids, viral vectors, cosmids, bacterial vectors and artificial chromosomes.

### Method for manufacturing enantiomerically pure (13R)-manoyl-oxide

In one aspect, the invention relates to a method of manufacturing substantially pure (13R)-manoyl oxide, said method comprising the steps of:
(i) providing geranylgeranyl diphosphate (GGPP);
(ii) contacting GGPP of step (i) with a first polypeptide having a sequence at least 75% identical to SEQ ID NO: 1 [CfTPS2] or SEQ ID NO: 2 [SsLPPS], thus obtaining labd-13-en-8,15-diol diphosphate (LPP);
(iii) contacting the LPP of step (ii) with a second polypeptide having a sequence at least 75% identical to SEQ ID NO: 3 [CfTPS4], SEQ ID NO: 4 [CfTPS3] or SEQ ID NO: 5 [EpTPS8]; thus obtaining (13R)-manoyl-oxide.

Manoyl oxide is a compound of general formula C₂₀H₃₄O. It has been detected as an experimental artefact in a racemic mixture of the (13R) epimer (I) and (13S) epimer (II). 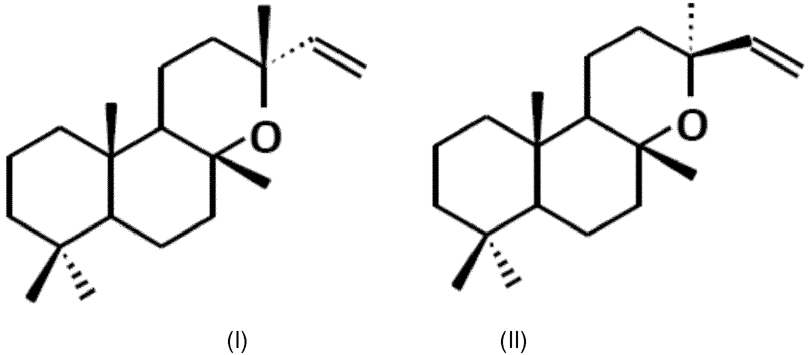 (13R)-manoyl oxide has been hypothesised to be a precursor for the synthesis of forskolin.

The inventors have found that (13R)-manoyl oxide may be synthesised stereospecifically, i.e. in an enantiomerically substantially pure form, by the following reaction: 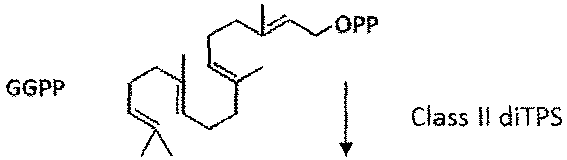 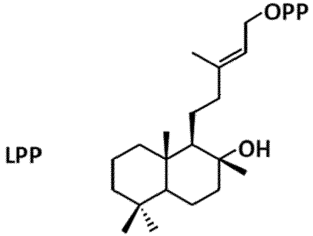 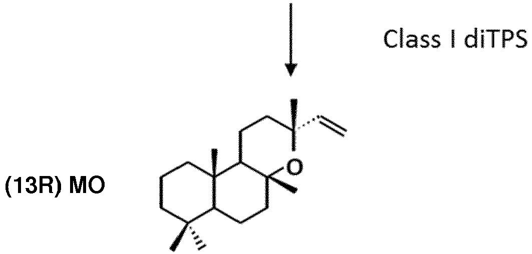 where GGPP is geranylgeranyl diphosphate, LPP is labd-13-en-8,15-diol diphosphate, and (13R) MO is (13R)-manoyl oxide.

Racemic mixtures of manoyl oxide have been disclosed, however it is very difficult to separate an enantiomerically pure (13R)-MO from such a racemic mixture. Thus, currently no established methods for purifying large amounts (e.g. gram scales) of enantiomerically pure (13R)-manoyl oxide are available.

The first step of the reaction is catalysed by a diterpene synthase (diTPS) having a class II diTPS activity. The class II diTPS catalyses protonation-initiated cationic cycloisomerization of GGPP to LPP. The reaction is terminated either by deprotonation or by water capture of the diphosphate carbocation. The first step of the reaction may be catalysed by any of the Class II diTPS described herein below in the section "Class II diterpene synthase". Class II diTPS particularly relevant for the invention are TPS2 from *Coleus forskohlii* (CfTPS2) and LPPS from *Salvia sclarea* (SsLPPS). In the method of the invention, the polypeptides are as defined in claim 1.

The method of the invention allows manufacturing of (13R)-manoyl oxide. In some embodiments, the (13R)-manoyl oxide obtained is substantially pure. Thus in some embodiments, the (13R)-manoyl oxide is more than 90% pure, such as 91% pure, such as 92% pure, such as 93% pure, such as 94% pure, such as 95% pure, such as 96% pure, such as 97% pure, such as 98% pure, such as 99% pure, such as 100% pure. In preferred embodiments, the (13R)-manoyl oxide manufactured by the method of the invention is more than 95% pure. In a preferred embodiment, the (13R)-manoyl oxide is 99% pure. In another preferred embodiment, the (13R)-manoyl oxide is 100% pure. Also disclosed herein is a method for manufacturing (13R)-manoyl oxide which is enantiomerically pure. The manoyl-oxide manufactured by the method of the invention is essentially (13R)-manoyl oxide. The (13R)-manoyl oxide of the invention is more than 90% enantiomerically pure, such as 91% enantiomerically pure, such as 92% enantiomerically pure, such as 93% enantiomerically pure, such as 94% enantiomerically pure, such as 95% enantiomerically pure, such as 96% enantiomerically pure, such as 97% enantiomerically pure, such as 98% enantiomerically pure, such as 99% enantiomerically pure, such as 100% enantiomerically pure. In preferred embodiments, the (13R)-manoyl oxide manufactured by the method of the invention is more than 95% enantiomerically pure. In a preferred embodiment, the (13R)-manoyl oxide is 99% enantiomerically pure. In another preferred embodiment, the (13R)-manoyl oxide is 100% enantiomerically pure.

The product obtained by performing the method of the invention is essentially free of (13S)-manoyl oxide. In some embodiments, the product obtained comprises less than 10% (13S)-manoyl oxide, such as less than 9% (13S)-manoyl oxide, such as less than 8% (13S)-manoyl oxide, such as less than 7% (13S)-manoyl oxide, such as less than 6% (13S)-manoyl oxide, such as less than 5% (13S)-manoyl oxide, such as less than 4% (13S)-manoyl oxide, such as less than 3% (13S)-manoyl oxide, such as less than 2% (13S)-manoyl oxide, such as less than 1%(13S)-manoyl oxide, such as 0% (13S)-manoyl oxide. In a preferred embodiment, the product obtained comprises less than 1% (13S)-manoyl oxide. In another preferred embodiment, the product obtained comprises no (13S)-manoyl oxide.

The method of the invention is performed by contacting GGPP with a first polypeptide having a class II diTPS activity and a second polypeptide having a class I diTPS activity.

In one embodiment, the first polypeptide has a sequence at least 75% identical to, such as at least 80% identical to, such as at least 85% identical to, such as at least 90% identical to, such as at least 95% identical to, such as 100% identical to SEQ ID NO: 1 [CfTPS2].

In another embodiment, the first polypeptide has a sequence at least 75% identical to, such as at least 80% identical to, such as at least 85% identical to, such as at least 90% identical to, such as at least 95% identical to, such as 100% identical to SEQ ID NO: 2 [SsLPPS].

The second step of the reaction is catalysed by a diTPS having a class I diTPS activity. It catalyzes cleavage of the diphosphate group of LPP and additional cyclization or rearrangement reactions on the resulting carbocation, yielding (13R)-manoyl oxide. As with the class II diTPSs, deprotonation or water capture terminate the class I diTPS reaction. The second step of the reaction may be catalysed by any of the Class I diTPS described herein below in the section "Class I diterpene synthase". Class I diTPS particularly relevant for the invention are TSP3 and TPS4 from *Coleus forskohlii* (CfTPS3 and CfTPS4, respectively) and TPS8 from *Euphorbia peplus* (EpTPS8). In the method of the invention, the polypeptides are as defined in claim 1.

In one embodiment, the second polypeptide has a sequence at least 75% identical to, such as at least 80% identical to, such as at least 85% identical to, such as at least 90% identical to, such as at least 95% identical to, such as 100% identical to SEQ ID NO: 3 [CfTPS4].

In another embodiment, the second polypeptide has a sequence at least 75% identical to, such as at least 80% identical to, such as at least 85% identical to, such as at least 90% identical to, such as at least 95% identical to, such as 100% identical to SEQ ID NO: 4 [CfTPS3].

In a further embodiment, the second polypeptide has a sequence at least 75% identical to, such as at least 80% identical to, such as at least 85% identical to, such as at least 90% identical to, such as at least 95% identical to, such as 100% identical to SEQ ID NO: 5 [EpTPS8].

In a preferred embodiment, the first polypeptide has a sequence at least 75% identical to, such as at least 80% identical to, such as at least 85% identical to, such as at least 90% identical to, such as at least 95% identical to, such as 100% identical to SEQ ID NO: 1 [CfTPS2] and the second polypeptide has a sequence at least 75% identical to, such as at least 80% identical to, such as at least 85% identical to, such as at least 90% identical to, such as at least 95% identical to, such as 100% identical to SEQ ID NO: 3 [CfTPS4]. In one embodiment, the first polypeptide has a sequence identical to SEQ ID NO: 1 [CfTPS2] and the second polypeptide has a sequence identical to SEQ ID NO: 3 [CfTPS4].

For example, the first polypeptide may be a biologically active sequence variant of CfTPS2 of SEQ ID NO:1, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 1 [CfTPS2], and the second polypeptide may be a biologically active sequence variant of CfTPS4 of SEQ ID NO:3, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 3 [CfTPS4].

In a preferred embodiment, the first polypeptide has a sequence at least 75% identical to, such as at least 80% identical to, such as at least 85% identical to, such as at least 90% identical to, such as at least 95% identical to, such as 100% identical to SEQ ID NO: 1 [CfTPS2] and the second polypeptide has a sequence at least 75% identical to, such as at least 80% identical to, such as at least 85% identical to, such as at least 90% identical to, such as at least 95% identical to, such as 100% identical to SEQ ID NO: 4 [CfTPS3]. In one embodiment, the first polypeptide has a sequence identical to SEQ ID NO: 1 [CfTPS2] and the second polypeptide has a sequence identical to SEQ ID NO: 4 [CfTPS3].

Thus it is preferred that the first polypeptide may be a biologically active sequence variant of CfTPS2 of SEQ ID NO:1, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 1 [CfTPS2], and the second polypeptide may be a biologically active sequence variant of CfTPS3 of SEQ ID NO:4, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 4 [CfTPS3]. In particular, the first polypeptide may be CfTPS2 of SEQ ID NO:1 or a biologically active sequence variant of CfTPS2 of SEQ ID NO:1, wherein the sequence variant is at least 80% identical to, EQ ID NO: 1 [CfTPS2], and the second polypeptide may be CfTPS3 of SEQ ID NO:4 or a biologically active sequence variant of CfTPS3 of SEQ ID NO:4, wherein the sequence variant is at least 80% identical to SEQ ID NO: 4 [CfTPS3]. This may in particular be preferred in embodiments of the invention relating to methods for producing (13R)-manoyl oxide that is more than 95% enantiomerically pure.

In another embodiment, the first polypeptide has a sequence at least 75% identical to, such as at least 80% identical to, such as at least 85% identical to, such as at least 90% identical to, such as at least 95% identical to, such as 100% identical to SEQ ID NO: 1 [CfTPS2] and the second polypeptide has a sequence at least 75% identical to, such as at least 80% identical to, such as at least 85% identical to, such as at least 90% identical to, such as at least 95% identical to, such as 100% identical to SEQ ID NO: 5 [EpTPS8]. In one embodiment, the first polypeptide has a sequence identical to SEQ ID NO: 1 [CfTPS2] and the second polypeptide has a sequence identical to SEQ ID NO: 5 [EpTPS8].

In another embodiment, the first polypeptide has a sequence at least 75% identical to, such as at least 80% identical to, such as at least 85% identical to, such as at least 90% identical to, such as at least 95% identical to, such as 100% identical to SEQ ID NO: 2 [SsLPPS] and the second polypeptide has a sequence at least 75% identical to, such as at least 80% identical to, such as at least 85% identical to, such as at least 90% identical to, such as at least 95% identical to, such as 100% identical to SEQ ID NO: 3 [CfTPS4]. In one embodiment, the first polypeptide has a sequence identical to SEQ ID NO: 2 [SsLPPS] and the second polypeptide has a sequence identical to SEQ ID NO: 3 [CfTPS4].

In another embodiment, the first polypeptide has a sequence at least 75% identical to, such as at least 80% identical to, such as at least 85% identical to, such as at least 90% identical to, such as at least 95% identical to, such as 100% identical to SEQ ID NO: 2 [SsLPPS] and the second polypeptide has a sequence at least 75% identical to, such as at least 80% identical to, such as at least 85% identical to, such as at least 90% identical to, such as at least 95% identical to, such as 100% identical to SEQ ID NO: 4 [CfTPS3]. In one embodiment, the first polypeptide has a sequence identical to SEQ ID NO: 2 [SsLPPS] and the second polypeptide has a sequence identical to SEQ ID NO: 4 [CfTPS3].

In another embodiment, the first polypeptide has a sequence at least 75% identical to, such as at least 80% identical to, such as at least 85% identical to, such as at least 90% identical to, such as at least 95% identical to, such as 100% identical to SEQ ID NO: 2 [SsLPPS] and the second polypeptide has a sequence at least 75% identical to, such as at least 80% identical to, such as at least 85% identical to, such as at least 90% identical to, such as at least 95% identical to, such as 100% identical to SEQ ID NO: 5 [EpTPS8]. In one embodiment, the first polypeptide has a sequence identical to SEQ ID NO: 2 [SsLPPS] and the second polypeptide has a sequence identical to SEQ ID NO: 5 [EpTPS8].

In order to obtain enantiomerically pure (13R)-manoyl oxide it is preferred that the first and the second polypeptides are present at a stoichiometry that allows enantiomerically pure (13R)-manoyl oxide to be yielded by the reaction. The first and second polypeptides are preferably present in the reaction at a stoichiometry ratio between 2:1 and 1:2. Preferably, the first and the second polypeptides are present in equal amounts, i.e. in a stoichiometry 1.1. Other stoichiometry ratios may lead to unbalanced reactions, where the produced manoyl oxide is in a racemic mixture, where manoyl-oxide is present both in the form of (13R)-manoyl oxide and (13S)-manoyl oxide.
In some embodiments, the method of the invention further comprises a step of recovering (13R)-manoyl oxide by methods known in the art.

The method of the invention can be performed in vivo. The first and the second polypeptides may be heterologously expressed in a host organism by methods known in the art. The host organism may be a prokaryote or a eukaryote. In some embodiments, the host organism is selected from the group comprising bacteria, yeast, fungi, plants, insects and mammals. The host organism may be selected from the group comprising *Escherichia coli, Saccharomyces cerevisiae, Schizosaccharomyces pombe, Nicotiana benthamiana* and *Physcomitrella patens.* Thus in one embodiment, the first and the second polypeptides are heterologously expressed in *Escherichia coli.* In another embodiment, the first and the second polypeptides are heterologously expressed in *Saccharomyces cerevisiae.* Thus in yet another embodiment, the first and the second polypeptides are heterologously expressed in *Nicotiana benthamiana.* In a preferred embodiment, the first and the second polypeptides are heterologously expressed from *Nicotiana benthamiana.* Methods for expressing the first and the second polypeptide in a host organism are known to those skilled in the art.

For performing the method of the invention in vivo, the GGPP may be provided in a composition or it may be provided by the host organism or by a second host organism.

In some embodiments, the host organism is capable of secreting the first polypeptide and the second polypeptide. The GGPP is provided in a composition or is provided by the host organism or by a second host organism, capable of secreting the GGPP. Thus in some embodiments, the reaction occurs in a composition comprising the first and the second polypeptides secreted by the host organism and GGPP provided in the composition or secreted by the host organism or a second host organism. The second host organism may be selected from the group comprising *Escherichia coli, Saccharomyces cerevisiae, Schizosaccharomyces pombe, Nicotiana benthamiana* and *Physcomitrella patens.* Preferably, the second host organism is identical to the host organism capable of heterologously expressing the first and the second polypeptides. The method of the invention can also be performed in a host cell. The first and the second polypeptides may be heterologously expressed in the host cell by methods known in the art. The host cell may be a prokaryote or a eukaryote. In some embodiments, the host cell is selected from the group comprising bacteria, yeast, fungi, plants, insects and mammals. The host cell may be selected from the group comprising *Escherichia coli, Saccharomyces cerevisiae, Schizosaccharomyces pombe, Nicotiana benthamiana* and *Physcomitrella patens.* Thus in one embodiment, the first and the second polypeptides are heterologously expressed in *Escherichia coli.* In another embodiment, the first and the second polypeptides are heterologously expressed in *Saccharomyces cerevisiae.* In yet another embodiment, the first and the second polypeptides are heterologously expressed in *Nicotiana benthamiana.* In a preferred embodiment, the first and the second polypeptides are heterologously expressed from *Nicotiana benthamiana.* Methods for expressing the first and the second polypeptide in a host organism are known to those skilled in the art.

The starting substrate of the reaction being GGPP, it may be an advantage that the host cell is further capable of producing GGPP. Thus the host cell may be genetically engineered to be capable of synthesising GGPP. This can be performed by methods known in the art. In a preferred embodiment, the GGPP is produced via the plastidial methylerythritol 4-phosphate (MEP) pathway, which can be cloned in the host cell.

In other embodiments, the method of manufacturing (13R)-manoyl oxide is performed in vitro. In such embodiments, the first and the second polypeptides may be heterologously expressed from a host organism as described above, and subsequently purified before being contacted with GGPP provided in a composition. The GGPP may itself be produced by a host organism as detailed above.

The method of the invention may in some embodiments comprise a step of recovering the (13R)-manoyl oxide by methods known in the art. Such methods may involve solid-phase microextraction from plant leaves when the method is performed in a plant. (see for example Spanner et al., 2013). The host cell may also be capable of secreting (13R)-manoyl oxide, thereby facilitating its recovery.

The invention further relates to a non-enzymatic method for manufacturing forskolin using (13R)-manoyl oxide substrate, as defined in claim 12. In some embodiments, (13R)-manoyl oxide is converted via chemical reactions performed in vitro to produce forskolin. Such chemical reactions do not comprise enzymatic reactions. These non-enzymatic reactions are well known by those of skill in the art.

### Class I diterpene synthases

In one aspect the invention provides a polypeptide having diPTS activity. Said polypeptide is useful in the methods of manufacturing (13R)-manoyl oxide according to the invention,

The polypeptide of the invention, as defined in claim 14, has a diTPS activity. In some embodiments, the polypeptide has a class I diTPS activity. Class I diTPS are capable of cleaving diphosphate groups and performing rearrangement reactions such as cyclization.

Some polypeptides of the invention have a class I diTPS activity and catalyse cleavage of the diphosphate group of LPP and additional cyclization or rearrangement reactions on the resulting carbocation, yielding (13R)-manoyl oxide. Deprotonation or water capture terminate the class I diTPS reaction. Class I diTPS relevant for the invention are TSP3 and TPS4 from *Coleus forskohlii* (CfTPS3 and CfTPS4, respectively) and TPS8 from *Euphorbia peplus* (EpTPS8).

In one embodiment the polypeptide of the invention comprises:
i) an amino acid sequence identical to SEQ ID NO: 4 [CfTPS3],
ii) a biologically active sequence variant of said polypeptide, wherein the sequence variant is at least 90% identical to, such as at least 95% identical to, such as at least 96% identical to, such as at least 97% identical to, such as at least 98% identical to, such as at least 99% identical to, such as 100% identical to SEQ ID NO: 4 [CfTPS3].

In a preferred embodiment, the polypeptide comprises a biologically active sequence variant having an amino acid sequence at least 95% identical to SEQ ID NO: 4 [CfTPS3]. In another preferred embodiment, the polypeptide comprises an amino acid sequence 100% identical to SEQ ID NO: 4 [CfTPS3].

In another embodiment the polypeptide of the invention comprises:
i) an amino acid sequence identical to SEQ ID NO: 3 [CfTPS4],
ii) a biologically active sequence variant of said polypeptide, wherein the sequence variant is at least 90% identical to, such as at least 95% identical to, such as at least 96% identical to, such as at least 97% identical to, such as at least 98% identical to, such as at least 99% identical to, such as 100% identical to SEQ ID NO: 3 [CfTPS4].

In a preferred embodiment, the polypeptide comprises a biologically active sequence variant having an amino acid sequence at least 95% identical to SEQ ID NO: 3 [CfTPS4]. In another preferred embodiment, the polypeptide comprises an amino acid sequence 100% identical to SEQ ID NO: 3 [CfTPS4].

In yet another embodiment, embodiment the polypeptide of the invention comprises:
i) an amino acid sequence identical to SEQ ID NO: 5 [EpTPS8]; or
ii) a biologically active sequence variant of said polypeptide, wherein the sequence variant is at least
90% identical to, such as at least 95% identical to, such as at least 96% identical to, such as at least 97% identical to, such as at least 98% identical to, such as at least 99% identical to, such as 100% identical to SEQ ID NO: 5 [EpTPS8].

In some embodiments, the polypeptide of the invention comprises an operative class I DDxxD (SEQ ID NO: 12) domain. Preferably, the polypeptides of the invention (in particular the Class I diTPS) or variants or fragments thereof as defined above comprise a DDxxD domain. This domain is found at positions 531-535 of SEQ ID NO: 3 [CfTPS4], 299-303 of SEQ ID NO: 4 [CfTPS3], 540-544 of SEQ ID NO: 5 [EpTPS8]. Other important functional domains of the class I diTPS polypeptides of the invention are Mg²⁺-binding sites, active site lid residues and substrate binding pockets. The relevant residues for each of SEQ ID NO: 4 [CfTPS3], SEQ ID NO: 3 [CfTPS4] and SEQ ID NO: 5 [EpTPS8] are listed in Table 1. Some embodiments of the invention concern polypeptides, such as Class I diTPS in which these residues or domains are not modified.
In some embodiments, the polypeptides of the invention comprise a plastidial target domain. The plastidial target domain is comprised in the domain ranging from positions 1 to 73 of SEQ ID NO: 3 [CfTPS4], 1 to 73 of SEQ ID NO: 4 [CfTPS3], 1 to 73 of SEQ ID NO: 5 [EpTPS8], respectively. Thus preferred polypeptides (e.g. Class I diTPS) comprise the plastidial target domain.

**Table 1. Important domains and residues for class I diTPS of the invention. The residues are indicated as Xn, where X is the one letter amino acid code and n the position of the residue in the relevant sequence, except for the plastidial targeting signal, where the two numbers indicate the positions of the first and last residue of the predicted domain.**

| | Plastidial targeting signal | Mg²⁺-binding sites | Active site lid residues | Substrate binding pockets |
|---|---|---|---|---|
| CfTPS3 (SEQ ID NO: 4) | 1-73 | N443, E451 | EKERKENTGNSV (449-460); GDEF (526-529) | R262 L271 H292 V294 L295 T297 D300 D304 R440 L441 N443 D444 T446 E451 Y523 G526 F529 |
| CfTPS4 (SEQ ID NO: 3) | 1-73 | N475, E483 | EKERKENTGNSV (481-492); GDEF (559-562) | R294 L303 H324 V326 L327 T329 D332 D336 R472 L473 N475 D476 T478 E483 Y556 G559 F562 |
| EpTPS8 (SEQ ID NO: 5) | 1-73 | N684, E692 | KRESAQGKLNGV (690-701); DDGF (536-538) | R503 T512 N533 V535 F536 T538 D541 D545 R681 L682 N684 D685 T687 E692 Y764 D767 F770 |

| | | | | |
|---|---|---|---|---|
| Mg²⁺-binding sites for CfTPS3 (SEQ ID NO: 4) are found at positions 443 (N) and 451 (E). | | | | |

A biologically active sequence variant of a class I diTPS is preferably a polypeptide sharing the above mentioned sequence identity with CfTPS3, CfTPS4 or EpTPS8 and which preferably comprises above-mentioned domains and which is capable of catalysing cleavage of the diphosphate group of LPP and additional cyclization or rearrangement reactions on the resulting carbocation, yielding (13R)-manoyl oxide.

### Class II diterpene synthases

In one aspect the invention provides a polypeptide having diPTS activity. Said polypeptide is useful in the methods of manufacturing (13R)-manoyl oxide according to the invention,

In some embodiments, the polypeptide has a class II diTPS activity. Class II diTPS are capable of catalysing protonation-initiated cationic cycloisomerization reactions. The reaction is terminated either by deprotonation or by water capture of the diphosphate carbocation. A class II diTPS relevant for the invention is TPS2 from *Coleus forskohlii* (CfTPS2), which can catalyse cycloisomerisation of GGPP to LPP. Deprotonation or water capture terminate the class II diTPS reaction.

In one embodiment the polypeptide of the invention comprises:
i) an amino acid sequence identical to SEQ ID NO: 1 [CfTPS2],
ii) a biologically active sequence variant of said polypeptide, wherein the sequence variant is at least 90% identical to, such as at least 95% identical to, such as at least 96% identical to, such as at least 97% identical to, such as at least 98% identical to, such as at least 99% identical to, such as 100% identical to SEQ ID NO: 1 [CfTPS2].

In a preferred embodiment, the polypeptide comprises a biologically active sequence variant having an amino acid sequence at least 95% identical to SEQ ID NO: 1 [CfTPS2]. In another preferred embodiment, the polypeptide comprises an amino acid sequence 100% identical to SEQ ID NO: 1 [CfTPS2].

In some embodiments, the polypeptide of the invention comprises an operative class II DxDD domain. Preferably, the polypeptides of the invention (e.g. the Class II diTPS) or variants or fragments thereof as defined above comprise a DxDD domain. This domain is found at positions 358-361 of SEQ ID NO: 1 [CfTPS2].

In some embodiments, the polypeptides of the invention comprise a plastidial target domain. Thus, Class II diTPS may comprise a plastidial target domain. The plastidial target domain is comprised in the domain ranging from positions 1 to 73 of SEQ ID NO: 1 [CfTPS2].

A biologically active sequence variant of a class II diTPS is preferably a polypeptide sharing the above mentioned sequence identity with CfTPS2 or SsLPPS and which preferably comprises above-mentioned domains and which is capable of catalysing cycloisomerisation of GGPP to LPP.

### Polynucleotide

The invention further relates to a polynucleotide encoding a polypeptide according to the invention.

In some embodiments, the polynucleotide has a sequence with at least 90% identity to a sequence selected from the group consisting of SEQ ID NO: 6 [CfTPS2], SEQ ID NO: 9 [CfTPS3], SEQ ID NO: 8 [CfTPS4] and SEQ ID NO: 10 [EpTPS8].

In some embodiments, the polynucleotide has a sequence with at least 95% identity, such as at least 96% identity, such as at least 97% identity, such as at least 98% identity, such as 99% identity, such as 100% identity to SEQ ID NO: 6 [CfTPS2]. The polynucleotide may in particular encode a polypeptide of SEQ ID NO:1 [CfTPS2] or a biologically active sequence variant thereof sharing at least least 90%, such as at least 95%, such as at least 98% sequence identity with SEQ ID NO:1.

In other embodiments, the polynucleotide has a sequence with at least 90% identity, such as at least 95% identity, such as at least 96% identity, such as at least 97% identity, such as at least 98% identity, such as 99% identity, such as 100% identity to SEQ ID NO: 9 [CfTPS3]. The polynucleotide may in particular encode a polypeptide of SEQ ID NO:4 [CfTPS3] or a biologically active sequence variant thereof sharing at least 90%, such as at least 95%, such as at least 98% sequence identity with SEQ ID NO:4.

In other embodiments, the polynucleotide has a sequence with at least 90% identity, such as at least 95% identity, such as at least 96% identity, such as at least 97% identity, such as at least 98% identity, such as 99% identity, such as 100% identity to SEQ ID NO: 8 [CfTPS4]. The polynucleotide may in particular encode a polypeptide of SEQ ID NO:3 [CfTPS4] or a biologically active sequence variant thereof sharing at least 90%, such as at least 95%, such as at least 98% sequence identity with SEQ ID NO:3.

In other embodiments, the polynucleotide has a sequence with at least 90% identity, such as at least 95% identity, such as at least 96% identity, such as at least 97% identity, such as at least 98% identity, such as 99% identity, such as 100% identity to SEQ ID NO: 10 [EpTPS8]. The polynucleotide may in particular encode a polypeptide of SEQ ID NO:5 [EpTPS8] or a biologically active sequence variant thereof sharing at least 90%, such as at least 95%, such as at least 98% sequence identity with SEQ ID NO:5.
Thus the polynucleotides of the invention encode polypeptides having a diTPS activity as described above. Preferably, the polynucleotide comprises a sequence coding for an operative class I domain DDxxD. This is in particular the case, when the polynucleotide encode a class I diTPS. The DDxxD domain of the CfTPS3 diTPS is DDFFD, where D is an aspartic acid and F is a phenylalanine, and is found at positions 330 to 334 (SEQ ID NO: 4). The DDxxD domain of the CfTPS4 diTPS is DDFFD, where D is an aspartic acid and F is a phenylalanine, and is found at positions 331 to 335 (SEQ ID NO: 3). The DDxD domain of the EpTPS8 diTPS is DDFFD, where D is an aspartic acid and F is a phenylalanine, and is found at positions 540 to 544 (SEQ ID NO: 5).

In another preferred embodiment, the polynucleotide comprises a sequence coding for an operative class II domain DxDD. The DxDD domain of the CfTPS2 diTPS is DIDD, where D is an aspartic acid and I is an isoleucine residue, and is found at positions 399 to 402 (SEQ ID NO: 1).

Some polynucleotides of the invention may comprise a sequence coding for a plastidial targeting signal. The plastidial target domain is comprised in the corresponding polypeptides in the domains ranging from positions 1 to 50 of SEQ ID NO: 1 [CfTPS2], 1 to 33 of SEQ ID NO: 3 [CfTPS4], 1 to 3 of SEQ ID NO: 4 [CfTPS3], 1 to 5 of SEQ ID NO: 5 [EpTPS8], respectively.
The polynucleotide may have a sequence that is codon-optimised. Codon optimisation methods are known in the art and allow optimised expression in a heterologous host organism or cell. The host cell may be selected from the group comprising bacterial cell, yeast cells, fungal cells, plant cells, mammalian cells and insect cells. The host cell may be selected from the group comprising *Escherichia coli, Saccharomyces cerevisiae,Schizosaccharomyces pombe, Nicotiana benthamiana* and *Physcomitrella patens.* Thus in one embodiment, the host cell is *Escherichia coli.* In another embodiment, the host cell is *Saccharomyces cerevisiae* or *Schizosaccharomyces pombe.* In another embodiment, the host cell is *Nicotiana benthamiana.* In another embodiment, the host cell is *Physcomitrella patens.*

### Vectors

The invention further relates to a vector comprising at least one polynucleotide as defined above. Thus the invention relates to a vector suitable for expression of at least one polypeptide having a diTPS activity.

In some embodiments, the vector comprises a polynucleotide having a sequence with at least 85%
identity to a sequence selected from the group consisting of SEQ ID NO: 6 [CfTPS2], SEQ ID NO: 9 [CfTPS3], SEQ ID NO: 8 [CfTPS4] and SEQ ID NO: 10 [EpTPS8]. In some embodiments, the vector comprises a polynucleotide having a sequence with at least 85% identity, such as at least 90% identity, such as at least 95% identity, such as at least 96% identity, such as at least 97% identity, such as at least 98% identity, such as 99% identity, such as 100% identity to SEQ ID NO: 6 [CfTPS2]. In other embodiments, the vector comprises a polynucleotide having a sequence with at least 85% identity, such as at least 90% identity, such as at least 95% identity, such as at least 96% identity, such as at least 97% identity, such as at least 98% identity, such as 99% identity, such as 100% identity to SEQ ID NO: 9 [CfTPS3]. In other embodiments, the vector comprises a polynucleotide having a sequence with at least 85% identity, such as at least 90% identity, such as at least 95% identity, such as at least 96% identity, such as at least 97% identity, such as at least 98% identity, such as 99% identity, such as 100% identity to SEQ ID NO: 8 [CfTPS4]. In other embodiments, the polynucleotide has a sequence with at least 85% identity, such as at least 90% identity, such as at least 95% identity, such as at least 96% identity, such as at least 97% identity, such as at least 98% identity, such as 99% identity, such as 100% identity to SEQ ID NO: 10 [EpTPS8].

In some embodiments, the vector comprises a first polynucleotide having a sequence with at least 85%
identity to a sequence selected from the group consisting of SEQ ID NO: 6 [CfTPS2], SEQ ID NO: 9 [CfTPS3], SEQ ID NO: 8 [CfTPS4] and SEQ ID NO: 10 [EpTPS8] and a second polynucleotide having a sequence with at least 85%
identity to
   a sequence selected from the group consisting of SEQ ID NO: 6 [CfTPS2], SEQ ID NO: 9 [CfTPS3], SEQ ID NO: 8 [CfTPS4] and SEQ ID NO: 10 [EpTPS8], where the second polynucleotide is different from the first polynucleotide. In preferred embodiments, the vector comprises a first polynucleotide coding for a polypeptide with a class I diTPS activity and a second polynucleotide coding for a polypeptide with a class II diTPS activity. In a preferred embodiment, the first polynucleotide has a sequence with at least 85%
identity to SEQ ID NO: 6 [CfTPS2], and the second polynucleotide has a sequence with at least 85%
identity to SEQ ID NO: 9 [CfTPS3], SEQ ID NO: 8 [CfTPS4] and SEQ ID NO: 10 [EpTPS8].
   Vectors of the invention comprise plasmids, cosmids, viral vectors, artificial chromosomes and bacterial vectors. The vector may be suitable for transient expression of the at least one polynucleotide. Such vectors are known in the art. For example, expression may be induced by addition of a compound to the growth medium. The vector may be a bacterial vector, such as *Agrobacterium tumefaciens.* In a preferred embodiment, the vector also encodes a viral suppressor of gene silencing, such as the p19 protein of tomato bushy stunt virus.

### Host cell

The invention further relates to a host cell comprising a polynucleotide as defined in the claims.

The host cell of the invention is capable of expressing:
(i) a first polypeptide having a sequence at least 70% identical to SEQ ID NO: 1 [CfTPS2]; and
(ii) a second polypeptide having a sequence at least 70% identical to SEQ ID NO: 4 [CfTPS3], SEQ ID NO: 3 [CfTPS4], or SEQ ID NO: 5 [EpTPS8].

In some embodiments, the host cell is capable of expressing a first polypeptide having a class I diTPS activity and a second polypeptide having a class II diTPS activity.

Thus in some embodiments the host cell is capable of expressing a first polypeptide having a sequence at least 70% identical to SEQ ID NO: 1 [CfTPS2] and a second polypeptide having a sequence at least 70% identical to SEQ ID NO: 4 [CfTPS3]. In particular, the host cell is capable of expressing a first polypeptide, which is CfTPS2 of SEQ ID NO:1 or a biologically active variant thereof sharing at least 75%, such as at least 85%, such as at least 95% sequence identity with SEQ ID NO: 1 [CfTPS2] and a second polypeptide, which is CfTPS3 of SEQ ID NO:4 or a biologically active variant thereof sharing at least 75%, such as at least 85%, such as at least 95% sequence identity with SEQ ID NO:4 [CfTPS3].

In other embodiments, the host cell is capable of expressing a first polypeptide having a sequence at least 70% identical to SEQ ID NO: 1 [CfTPS2] and a second polypeptide having a sequence at least 70% identical to SEQ ID NO: 3 [CfTPS4]. In particular, the host cell is capable of expressing a first polypeptide, which is CfTPS2 of SEQ ID NO:1 or a biologically active variant thereof sharing at least 75%, such as at least 85%, such as at least 95% sequence identity with SEQ ID NO: 1 [CfTPS2] and a second polypeptide, which is CfTPS4 of SEQ ID NO:3 or a biologically active variant thereof sharing at least 75%, such as at least 85%, such as at least 95% sequence identity with SEQ ID NO:3 [CfTPS4].

In other embodiments, the host cell is capable of expressing a first polypeptide having a sequence at least 70% identical to SEQ ID NO: 1 [CfTPS2] and a second polypeptide having a sequence at least 70% identical to SEQ ID NO: 5 [EpTPS8]. In particular, the host cell is capable of expressing a first polypeptide, which is CfTPS2 of SEQ ID NO:1 or a biologically active variant thereof sharing at least 75%, such as at least 85%, such as at least 95% sequence identity with SEQ ID NO: 1 [CfTPS2] and a second polypeptide, which is EpTPS8 of SEQ ID NO:5 or a biologically active variant thereof sharing at least 75%, such as at least 85%, such as at least 95% sequence identity with SEQ ID NO:5 [EpTPS8].

The host cell may be selected from the group comprising bacterial cell, yeast cells, fungal cells, plant cells, mammalian cells and insect cells. The host cell may be selected from the group comprising *Escherichia coli, Saccharomyces cerevisiae, Schizosaccharomyces pombe, Nicotiana benthamiana* and *Physcomitrella patens.* Thus in one embodiment, the host cell is *Escherichia coli.* In another embodiment, the host cell is *Saccharomyces cerevisiae* or *Schizosaccharomyces pombe.* In another embodiment, the host cell is *Nicotiana benthamiana.* In another embodiment, the host cell is *Physcomitrella patens.*

Methods for expressing the first and second polypeptides in the host cell are known in the art. One or both of the first and second polypeptides may be heterologously expressed from polynucleotide sequences cloned into the genome of the host cell or they may be comprised within a vector as described above. For example, a first polynucleotide coding for the first polypeptide is cloned into the genome, and a second polynucleotide coding for the second polypeptide is comprised within a vector transformed or transfected into the host cell. Alternatively, the first polynucleotide is comprised within a first vector and the second polynucleotide is comprised within a second vector. The first and second vector may be one vector. Vectors suitable for expression of the first and second polypeptides are known in the art.

Expression of the first and second polypeptides in the host cell may occur in a transient manner. When the polynucleotide encoding one of the polypeptides is cloned into the genome, an inducible promoter may be cloned as well to control expression of the polypeptides. Such inducible promoters are known in the art. Alternatively, genes coding for suppressors of gene silencing may also be cloned in the genome or into a vector transfected within the host cell.

The host cell may be selected from the group comprising bacterial cell, yeast cells, fungal cells, plant cells, mammalian cells and insect cells. The host cell may be selected from the group comprising *Escherichia coli, Saccharomyces cerevisiae, Schizosaccharomyces pombe, Nicotiana benthamiana* and *Physcomitrella patens.* Thus in one embodiment, the host cell is *Escherichia coli.* In another embodiment, the host cell is *Saccharomyces cerevisiae* or *Schizosaccharomyces pombe.* In another embodiment, the host cell is *Nicotiana benthamiana.* In another embodiment, the host cell is *Physcomitrella patens.*

In some embodiments, the host cell is transfected with a vector. The vector may be selected from the group comprising plasmids, cosmids, viral vectors, artificial chromosomes and bacterial vectors. The vector may be constructed so that transient expression of the first and/or second polynucleotide from the vector is transient. Transient expression from the vector may occur via induction of an inducible promoter as is known in the art. In a preferred embodiment, the host cell is a *Nicotiana benthamiana* cell, such as a leaf cell. In another preferred embodiment, the vector is the bacterial vector *Agrobacterium tumefaciens.* In a preferred embodiment, the vector also encodes a viral suppressor of gene silencing. In a most preferred embodiment, the vector encodes the p19 protein from tomato bushy stunt virus.

The host cell may naturally be capable of producing GGPP. In other embodiments, the cell may be engineered so that it is capable of producing GGPP. For example, the plastidial methylerythritol 4-phosphate (MEP) pathway may be cloned into the host cell. Thus, the host cell may comprise one or more heterologous nucleic acids encoding one or more enzymes of the MEP pathway.

The host cell may be further engineered in order to redirect metabolic fluxes and optimise for the production of a specific compound.

In some embodiments, the host cell is capable of producing diterpenoids. The host cell may be capable of producing (13R)-manoyl oxide in a substantially pure enantiomeric form.

### Examples

### Example 1: Identification of Coleus forskohlii diTPS genes.

Forskolin is a representative of an unusual series of tricyclic (8,13)-epoxy-labdanes, characteristic for this plant. Given its importance as a pharmaceutical, we set out to discover genes involved in the biosynthesis of forskolin.

### Materials and methods

### Plant Growth and Microscopy

*Coleus forskohlii* (Lamiaceae) plants were grown in the greenhouse at the University of Copenhagen, Denmark, under ambient photoperiod and 24°C day/17°C night temperatures. Transverse sections of roots (diameter of approximately 1 to 5 mm) were prepared for histochemical analysis. Sections were observed unstained with a Leica DM 5000B or a Nikon Eclipse 80i light and fluorescence microscope.

Additionally, root samples were fixed in a solution containing 2.5% glutaraldehyde, 2% paraformaldehyde, and 0.1 M sodium cacodylate buffer, pH 7.2 for 24 h, thereafter surface sections and cross sections from the root cork were incubated in 0.1 µg/mL Nile Red for identification of lipid components. Images from intact cells were recorded in a Leica SP5X confocal laser scanning microscope (Leica Microsystems, Mannheim, Germany).

### Diterpene profiling and forskolin quantification in C. forskohlii tissues

Tissue was extracted. Cold methanol acidified with formic acid (0.125%) was added to ground and frozen tissue samples in a ratio of 3:1 (solvent:tissue). Samples were sonicated in an ultrasonic bath at 23°C for 15 min at 40 kHz (Branson, 3510), filtered using 96 well filter plates and analyzed by HPLC (High-Performance Liquid Chromatography) equipped with an evaporative light scattering detector (ELSD). All tissue types were extracted in triplicate. Forskolin was quantified by comparison to a standard series of forskolin (Sigma) which was prepared as per the tissue samples.

For the diterpene profiling of the root cork HPLC-MS, extracts were prepared as for forskolin quantification. Separation was carried out on an Agilent 1100 Series LC unit (Agilent Technology) with column and gradient as described above. The LC unit was coupled to either a Bruker microTOF mass spectrometer (Bruker Daltronics) for high resolution mass measurements or a Bruker HCT-Ultra ion trap mass spectrometer (Bruker Daltronics) for MSn experiments.

### Isolation of C. forskohlii root cork oil bodies

For isolation of oil bodies from root cork tissue, approximately 15 g of tissue was gently ground in 100 mL extraction buffer (EB; 20 mM Tricine, 250 mM sucrose, 0.2 mM PMSF, pH 8.5); the homogenate was filtered through Miracloth (Calbiochem) and centrifuged at 3500 rpm for 10 min for separation of cellular debris. The supernatant was collected and transferred in centrifugation tubes. Buffer B (20% sucrose, 20 mM HEPES, 100 mM KCI, 2 mM MgCl₂, pH 10.5) was overlaid (5 mL of B for 25 mL of supernatant) and samples were centrifuged for 40 min at 5000 g. The resulting floating oil bodies were collected carefully from the surface layer.

### Identification and cloning of full length diTPS genes

Mining of the *C. forskohlii* databases was performed as previously described (Zerbe et al., 2013), using tBLASTn software and known angiosperm diTPSs as query (CPS and EKS) and guided full-length cloning of a number of putative class I and class II diTPS genes. Total RNA from *C. forskohlii* roots, extracted as previously described (Hamberger et al., 2011), was used for cDNA synthesis. Cloning of the putative diTPS genes was achieved after PCR amplification using gene specific primers (SEQ ID NO: 13 to 42) that were designed based on the *in silico* sequences of the identified CfTPS genes.

### RNA extraction and Quantitative Real-Time PCR

Total RNA from *C. forskohlii* root cork was extracted according to Hamberger et al., 2011, and further purified using the Spectrum Plant Total RNA Kit (Sigma) while total RNA from leaves, flowers, stems and root cortex and stele was extracted using the Spectrum Plant Total RNA Kit (Sigma). RNA extraction was followed by on-column DNase I digestion. First-strand cDNAs were synthesized from 0.5 µg of total RNA. The resulting cDNA was diluted 10-fold for the qRT-PCR reactions. Quantitative real-time PCR reactions were performed with gene specific primers (SEQ ID NO: 43 to 62) and Maxima SYBR Green/Fluorescein qPCR Master Mix (Fermentas) on a Rotor-Gene Q cycler (Qiagen). TIF4a and EF1a were used as reference genes as they showed the lowest variation across different tissues. The results were normalized with TIF4a. Relative transcript abundance was calculated as the mean of three biological replications (three different plants), while the reactions were performed in three technical replicates. Amplification efficiency was calculated with the "Real Time PCR Miner" (http://www.miner.ewindup.info/Version2). Efficiency-corrected ΔC_{T} values were used to quantify relative differences in target gene transcript accumulation.

### Functional characterization of CfTPS - in vitro assays

For the expression of CfTPS1, CfTPS2, CfTPS3, CfTPS4 and CfTPS14 in *E. coli,* pseudomature variants lacking predicted plastidial target sequences were cloned into the into pET28b+ vector. The software ChloroP was used for prediction of the plastidial target sequence (http://www.cbs.dtu.dk/services/ChloroP/). As the expression levels of the recombinant CfTPS3 were very poor, a codon optimized version was synthesized by GenScript USA Inc. and subsequently cloned into the same vector (SEQ ID NO: 63). pET28b+ constructs were transformed into *E*. *coli* BL-21DE3-C41 cells and and grown in selection medium until the OD₆₀₀ reached 0.3-0.4. Expression was induced at OD₆₀₀ ∼0.6 with 0.2 mM IPTG. Expression was done overnight and cells were harvested by centrifugation and lysed. The cell lysates were centrifuged and the supernatant was subsequently used for purification of the recombinant proteins. CfTPS proteins were purified on 1 mL His SpinTrap™ columns (GE healthcare). *In vitro* CfTPS assays were performed by adding 15 µM GGPP and 100 µg purified CfTPS enzymes in 397 µL enzyme assay buffer (50 mM HEPES (pH 7.2), 7.5 mM MgCl₂, 5% (v/v) glycerol, 5 mM DTT). Onto the reaction mix, 500 µL n-hexane (Fluka GC-MS grade) was gently added as an overlay. Assays were incubated for 60 min at 30°C and ∼70 rpm and the hexane overlay was subsequently removed for GC-MS analysis.

### Functional characterization of CfTPS - transient expression in Nicotiana benthamiana

Full length CfTPS cDNAs were cloned into the agrobacterium binary vector for plant transformation pCAMBIA1300_35Su. Transient expression of *CfTPSs* with the gene silencing suppressor p19 protein in *N. benthamiana* leaves and extraction of diterpenes were performed as recently described. Hexane extracts of *N. benthamiana* expressing the gene silencing suppressor p19 protein alone were used as controls. Compounds of interest were identified by comparison of GC-MS total ion chromatogram (TIC) and extracted ion chromatograms (EIC) of 275 and 272 m/z from samples. The ion 275 m/z is characteristic of several labdane type diterpenes including manoyl oxide whereas 272 m/z is characteristic of several other non-labdane type diterpenes such as abietane like diterpenes. All extractions from *N. benthamiana* transiently expressing diTPSs were carried out in biological triplicates (different leaves/plants infiltrated with the same agrobacteria mixture).

### Metabolite analysis from in vitro and in planta assays

For the gas chromatography-mass spectrometry (GC-MS) analysis of *N. benthamiana* leaves expressing the CfTPSs and specific *C*. *forskohlii* tissues, 500 µL GC-MS grade hexane was added to 2 leaf discs (Ø=3 cm) in a 1.5 mL glass vial. After extraction, the solvent was transferred into new 1.5 mL glass vials and stored at -20°C until GC-MS analysis. Compound identification was done by comparison to authentic standards (dehydroabietadiene, abietadiene), reference spectra from literature, databases and comparison of retention time (miltiradiene, manoyl oxide, copalol, labd-13-en-8,15-diol and 13(16)-14-labdien-8-ol) (Wiley Registry of Mass Spectral Data, 8th Edition, July 2006, John Wiley & Sons, ISBN: 978-0-470-04785-9). The differentiation of the C-13 epimers (13R) and (13S)-manoyl oxide was performed as previously described (Demetzos et al., 2002).

### Accession numbers

Nucleotide sequences of characterized enzymes have been submitted to the GenBankTM/EBI Data Bank with accession numbers: CfTPS1, KF444506; CfTPS2, KF444507; CfTPS3, KF444508; CfTPS4, KF444509; CfTPS15, KF471011.

### Example 2: Localization of forskolin and (13R)-manoyl oxide in root cork oil bodies

When transverse sections of *C. forskohlii* root (Fig. 1A) were examined using light microscopy, we found that cells of the root cork contained oil body-like structures (hereafter termed oil bodies) with a typical distribution of one oil body per cell of the root cork (Fig. 1B). Cells containing more than one oil body were occasionally seen in older tissue (Fig. 1C). Confocal laser scanning microscopy of *C. forskohlii* root cork stained with Nile Red indicated that the observed structures were indeed oil bodies and that the composition of the lipophilic content was heterogeneous, with both polar (Fig. 1E) and neutral (Fig. 1D) lipophilic compounds, which were non-uniformly distributed. Globules of neutral lipids dispersed in predominantly polar lipids were detected by the fluorescence (Fig. 1D-F).

Separate methanol extracts of the root cork and the root stele and cortex were analysed by high-performance liquid chromatography (HPLC) using an evaporative light scattering detector (ELSD) and compared with flowers, leaves and stems. Forskolin was primarily detected in the root cork, and was not found in the root stele and cortex nor in the aerial parts of the plant (Fig. 2). To further examine if forskolin was present specifically in the oil bodies, methanol extracts of isolated oil bodies (oil bodies purified to apparent homogeneity) were subjected to HPLC-ELSD analysis, targeting polar constituents, while non-polar hexane extracts were analyzed by gas chromatography-mass spectrometry (GC-MS) (Fig. 3). In addition to forskolin, which was present in the polar fraction, we detected (13R)-manoyl oxide in the non-polar fraction.

These results demonstrate that (13R)-manoyl oxide and forskolin are present in the oil bodies of *C. forskohlii.*

### Example 3: C. forskohlii diTPSs constitute a small gene family specific for Lamiaceae

We mined the root transcriptome of *C. forskohlii* for the identification of *CfdiTPS* candidates as described (Zerbe et al., 2013). A panel of six diterpene synthases was identified, *CfTPS1, CfTPS2, CfTPS3, CfTPS4, CfTPS14* and *CfTPS15* which, with exception of *CfTPS15,* represented full-length cDNAs with predicted N-terminal plastidial transit peptides. *CfTPS1, CfTPS2,* and *CfTPS15* contained the Asp-rich conserved motif DxDD characteristic of class II diTPS, while *CfTPS3, CfTPS4* and *CfTPS14* carried the DDxxD signature motif of class I diTPS. We performed separate phylogenetic analyses of class II and class I CfTPSs including functionally characterized representatives from the Lamiaceae and other angiosperm species. Included in the phylogenies were representative gymnosperm class II and class I diTPSs [PgCPS and PgEKS] and the bifunctional diTPS from the moss *Physcomitrella patens* [PpCPS/EKS], as it is considered an ancestral archetype of plant diTPSs (Fig. 4).

Thus we demonstrate that *CfTPS1, CfTPS2,* and *CfTPS15* are phylogenetically related to class II diTPS and that *CfTPS3, CfTPS4* and *CfTPS14* are phylogenetically related to class I diTPS.

### Example 4: Transcript levels of C. forskohlii diTPSs in various tissues and in vitro functional characterization

To correlate the transcript levels of *CfTPS* genes with accumulation of forskolin related labdane-diterpenoids and abietane diterpenoids in *C. forskohlii* tissues, quantitative (q)RT-PCR analysis was performed using cDNA templates derived from the root cork (Ck), root cortex and stele (root without cork) (CS), leaves (Lv), stems (St) and flowers (FI) total RNA. *CfTPS1, CfTPS2* and *CfTPS3* shared a similar pattern of transcript profiles across all tissues, showing high transcript accumulation in root cork cells, up to 1000-fold in comparison with all other tissues tested (Fig. 5). These data supported involvement of *CfTPS1, CfTPS2* and *CfTPS3* in the formation of specialized metabolites in the root cork. In contrast, the transcript levels of *CfTPS4, CfTPS14* and *CfTPS15* were relatively low across all tissues tested. Despite the close phylogenetic relation of *CfTPS3* and *CfTPS4* (Fig. 4), they show surprisingly different expression patterns. In contrast to *CfTPS3, CfTPS4* transcripts were mostly detected in the aerial parts of the plant, especially in the leaves, while its transcripts accumulate only to very low levels in the root.

For the functional characterization of the CfTPSs described here (except for CfTPS1 5, for which no full length sequence could be retrieved), cDNAs were heterologously expressed in *E. coli* with a C-terminal 6xHis epitope tag. Purified recombinant proteins were tested individually in single or coupled *in vitro* assays, supplied with appropriate substrates and the reaction products were analyzed by GC-MS. Products of the *in vitro* assays with the class II diTSPs, CfTPS1 and CfTPS2, were treated with alkaline phosphatase before GC-MS analysis.

Enzyme assays with CfTPS1 yielded a diterpene with a mass spectrum matching copal-15-ol (h), indicating that the primary product before dephosphorylation is copalyl diphosphate (Fig. 6A). Assays of CfTPS2 resulted in the formation of 13(16)-14-labdien-8-ol (f) and labd-13-en-8,15-diol (g) as major products (Fig. 6B), supporting a function as labda-13-en-8-ol (or copal-8-ol) diphosphate synthase, similar to the functions of previous reported similar enzymes. We also detected the non-stereoselective formation of the (13R) and (13S) epimers of manoyl oxide, which were previously observed in *in vitro* reactions of similar class II diTPSs and were suggested to be the result of a non-enzymatic reaction (Caniard et al., 2012; Zerbe et al., 2013). These results indicate that CfTPS1 and CfTPS2 represent functionally distinct class II diTPSs, both necessary and sufficient to form the diphosphate intermediates required for the abietane and labdane classes of diterpenoids detected in *C. forskohlii.*

Assays of CfTPS1 coupled to either CfTPS3 or CfTPS4 resulted in formation of miltiradiene (d) (Fig. 6A), a labdane diterpene formed from a copalyl diphosphate intermediate (Gao et al., 2009) and is consistent with the results of the single enzyme assay of CfTPS1. Coupled assays with CfTPS2 and CfTPS3 showed the formation of the pure (13R) enantiomer of manoyl oxide (a) (Fig. 6B). In coupled assays of CfTPS2 with CfTPS4, both (13R) and (13S)-manoyl oxide epimers were detected, albeit at a ratio different from the dephosphorylation product of CfTPS2 alone (Fig. 6B). The (13R)-manoyl oxide epimer was produced stereospecifically in the combination of class II CfTPS2 and class I CfTPS3 enzymes.

These results demonstrate that the CfTPSs can be expressed heterologously in *Escherichia coli.* We further show that (13R)-manoyl oxide can be produced stereospecifically in a substantially pure enantiomeric form in an *in vitro* system.

### Example 5: In planta heterologous expression and functional characterization of C. forskohlii diTPSs

The CfTPSs were expressed in *Nicotiana benthamiana* leaves after agroinfiltration. GC-MS analyses of extracts from *N. benthamiana* leaves transiently expressing the individual class I CfTPS3, CfTPS4 and CfTPS14 did not result in detectable accumulation of additional metabolites compared to control plants (data not shown). Extracts from *N. benthamiana* expressing the class II CfTPS1 alone showed only trace amounts of additional diterpenes compared to the controls, none of which could be accurately identified (Fig. 7B).

Consistent with the *in vitro* enzyme assays, both (13R) and (13S) epimers of manoyl oxide were identified in the extracts from *N. benthamiana* expressing the class II CfTPS2 (Fig. 7A). Co-expression of CfTPS2 and CfTPS14 did not change the product profile compared to expression of CfTPS2 alone, suggesting that CfTPS14 does not accept the copal-8-ol diphosphate as substrate (Fig. 7A).

In extracts of plants co-expressing CfTPS1 with CfTPS3 or CfTPS4 miltiradiene (d) was observed as the main product together with minor traces of dehydroabietadiene (c) and abietadiene (e) (Fig. 7B). All three diterpenes were subsequently identified in stem and root tissues of *C. forskohlii.* While dehydroabietadiene was found in both tissues, abietadiene was mainly detected in the root cork tissue, and miltiradiene in the stem and leaf tissue of *C. forskohlii.*

In extracts from *N. benthamiana* co-expressing CfTPS2 with CfTPS3 or CfTPS4, only the (13R) epimer of manoyl oxide (a) was identified (Fig. 7A), consistent with the stereochemical conformation of forskolin and the related series of labdane-type diterpenoids. This result suggests that the class I CfTPS3 and CfTPS4 can accept the copal-8-ol diphosphate synthesized by CfTPS2 and catalyze the stereospecific formation of (13R)-manoyl oxide.

These results demonstrate that the CfTPSs can be agroinfiltrated in a plant organism and that (13R)-manoyl oxide can be produced stereospecifically in a substantially pure enantiomeric form in an *in vivo* system.

### References

Caniard et al., 2012. Discovery and functional characterization of two diterpene synthases for sclareol biosynthesis in Salvia sclarea (L.) and their relevance for perfume manufacture. BMC Plant Biol. 12:119.
Demetzos et al., 2002. A simple and rapid method for the differentiation of C-13 manoyl oxide epimers in biologically important samples using GC-MS analysis supported with NMR spectroscopy and computational chemistry results. Bioorg Med Chem Lett. 12(24):3605-9.
Günnewich et al., 2013. A diterpene synthase from the clary sage Salvia sclarea catalyzes the cyclization of geranylgeranyl diphosphate to (8R)-hydroxy-copalyl diphosphate. Phytochemistry 91:93-9
Hamberger et al., 2011. Evolution of diterpene metabolism: Sitka spruce CYP720B4 catalyzes multiple oxidations in resin acid biosynthesis of conifer defense against insects. Plant Physiol. 157(4):1677-95.
Zerbe et al., 2013. Gene discovery of modular diterpene metabolism in nonmodel systems. Plant Physiol. 162(2):1073-91.

## Claims

1. A method of manufacturing (13R)-manoyl oxide, comprising:
(i) providing geranylgeranyl diphosphate (GGPP);
(ii) contacting GGPP with a first polypeptide having at least 75% sequence identity to the amino acid sequence set forth in SEQ ID NO: 1 or SEQ ID NO: 2, thus obtaining labd-13-en-8,15-diol diphosphate (LPP);
(iii) contacting LPP with a second polypeptide having at least 75% sequence identity to the amino acid sequence set forth in SEQ ID NO: 3, SEQ ID NO: 4, or SEQ ID NO: 5;
thus producing (13R)-manoyl-oxide.

2. The method of claim 1, wherein the (13R)-manoyl oxide is enantiomerically pure.

3. The method of claim 1 or 2, wherein the first polypeptide and the second polypeptide are present at a ratio between 2:1 and 1:2.

4. The method of any one of claims 1 to 3, further comprising a step of recovering the (13R)-manoyl oxide.

5. The method of any one of claims 1 to 4, where the method is performed *in vivo.*

6. The method of any one of claims 1 to 5, wherein the first and the second polypeptides are heterologously expressed in a host organism is bacteria, yeast, fungi, plants or insects.

7. The method of any one of claims 1 to 6, wherein the host organism is *Escherichia coli, Saccharomyces cerevisiae, Schizosaccharomyces pombe, Nicotiana benthamiana* or *Physcomitrella patens.*

8. The method of any one of claims 1 to 7, wherein the first and the second polypeptides are purified after heterologous expression.

9. The method of any one of claims 1 to 8, wherein GGPP is provided in a composition or is produced by the host organism.

10. The method of any one of claims 1 to 9, wherein:
(a) the first polypeptide has at least 75% sequence identity to the amino acid sequence set forth in SEQ ID NO: 1; and
(b) the second polypeptide has at least 75% sequence identity to the amino acid sequence set forth in SEQ ID NO: 3.

11. The method of claim 9, wherein the host organism is capable of expressing the first and the second polypeptides at a ratio of 1:1.

12. The method of any one of claims 1 to 11, further comprising non-enzymatic synthesis of forskolin from (13R)-manoyl-oxide.

13. (13R)-manoyl oxide obtained by the method of any one of claims 1 to 12, wherein the (13R)-manoyl oxide is more than 90% enantiomerically pure.

14. An isolated diterpene synthase (diTPS) polypeptide of SEQ ID NO:1, 3, 4, or 5 or a biologically active variant thereof having at least 90% sequence identity to the amino acid sequence set forth in SEQ ID NO:1, 3, 4, or 5;
wherein the biological activity is diterpene synthase activity.

15. The polypeptide of claim 14, wherein the polypeptide has class I diTPS activity, and has a sequence that is at least 90% identical to the amino acid sequence set forth in SEQ ID NO:3, 4, or 5.

16. The polypeptide of claim 15, wherein the polypeptide is capable of catalysing cleavage of a diphosphate group of LPP and additional cyclization or rearrangement reactions on the resulting carbocation yielding (13R)-manoyl oxide.

17. The polypeptide of claim 14, wherein the polypeptide has class II diTPS activity, and has a sequence that is at least 90% identical to the amino acid sequence set forth in SEQ ID NO:1, wherein the polypeptide is capable of catalysing cycloisomerisation of GGPP to LPP.

18. The polypeptide of any one of claims 14 to 17, further comprising a plastidial targeting signal.

19. A polynucleotide encoding a polypeptide of any one of claims 14 to 18.

20. The polynucleotide of claim 19, wherein the polynucleotide has a sequence that is at least 85% identical to the nucleotide sequence set forth in SEQ ID NO:6, 8, 9, or 10.

21. The polynucleotide of any one of claims 19 or 20, further comprising a sequence coding for a plastidial targeting signal.

22. The polynucleotide of any one of claims 19 to 21, wherein the polynucleotide is codon-optimised for expression in a host cell that is a bacterial cell, a yeast cell, a fungal cell, a plant cell or an insect cell.

23. A vector comprising a polynucleotide of any one of claims 19 to 22.

24. A host cell comprising the polynucleotide of any one of claims 19 to 22, and/or the vector of claim 23.

25. The cell of claim 24, wherein the cell expresses:
(i) a first polypeptide of SEQ ID NO:1 or a biologically active variant thereof having at least 80% sequence identity to the amino acid sequence set forth in SEQ ID NO: 1; and
(ii) a second polypeptide of SEQ ID NO: 3, 4, or 5 or a biologically active variant thereof having at least 80% sequence identity to the amino acid sequence set forth in SEQ ID NO: 3, 4, or 5,
wherein the cell is a bacterial cell, a yeast cell, a fungal cell, a plant cell or an insect cell.

26. The cell of claim 25, wherein the cell is a *Nicotiana benthamiana* cell transfected with at least one viral vector for transiently expressing the first and the second polypeptides.

27. The cell of any one of claims 24 to 26, wherein the cell is further naturally capable of producing or further engineered to produce GGPP via the plastidial methylerythritol 4-phosphate (MEP) pathway.

28. The polypeptide of claim 14, wherein the polypeptide further comprises an operative class I DDxxD domain.

## Patentansprüche

1. Verfahren zur Herstellung von (13R)-Manoyloxid, das Folgendes umfasst:
(i) Bereitstellen von Geranylgeranyldiphosphat (GGPP);
(ii) Inkontaktbringen von GGPP mit einem ersten Polypeptid, das mindestens 75 % Sequenzidentität mit der Aminosäuresequenz, die in SEQ.-ID Nr. 1 oder SEQ.-ID Nr. dargelegt ist, aufweist, sodass Labd-13-en-8,15-dioldiphosphat (LPP) erhalten wird;
(iii) Inkontaktbringen von LPP mit einem zweiten Polypeptid, das mindestens 75 % Sequenzidentität mit der Aminosäuresequenz, die in SEQ.-ID Nr. 3, SEQ.-ID Nr. 4 oder SEQ.-ID Nr. 5 dargelegt ist, aufweist;
sodass (13R)-Manoyloxid erzeugt wird.

2. Verfahren nach Anspruch 1, wobei das (13R)-Manoyloxid enantiomerenrein ist.

3. Verfahren nach Anspruch 1 oder 2, wobei das erste Polypeptid und das zweite Polypeptid in einem Verhältnis zwischen 2 : 1 und 1 : 2 vorliegen.

4. Verfahren nach einem der Ansprüche 1 bis 3, das weiter einen Schritt der Gewinnung des (13R)-Manoyloxids umfasst.

5. Verfahren nach einem der Ansprüche 1 bis 4, wobei das Verfahren *in vivo* durchgeführt wird.

6. Verfahren nach einem der Ansprüche 1 bis 5, wobei das erste und das zweite Polypeptid in einem Wirtsorganismus heterolog exprimiert werden, der Bakterien, Hefe, Pilze, Pflanzen oder Insekten ist.

7. Verfahren nach einem der Ansprüche 1 bis 6, wobei der Wirtsorganismus *Escherichia coli, Saccharomyces cerevisiae, Schizosaccharomyces pombe, Nicotiana benthamiana* oder *Physcomitrella patens* ist.

8. Verfahren nach einem der Ansprüche 1 bis 7, wobei das erste und das zweite Polypeptid nach heterologer Expression aufgereinigt werden.

9. Verfahren nach einem der Ansprüche 1 bis 8, wobei GGPP in einer Zusammensetzung bereitgestellt wird oder von dem Wirtsorganismus erzeugt wird.

10. Verfahren nach einem der Ansprüche 1 bis 9, wobei:
(a) das erste Polypeptid mindestens 75 % Sequenzidentität mit der Aminosäuresequenz, die in SEQ.-ID Nr. 1 dargelegt ist, aufweist; und
(b) das zweite Polypeptid mindestens 75 % Sequenzidentität mit der Aminosäuresequenz, die in SEQ.-ID Nr. 3 dargelegt ist, aufweist.

11. Verfahren nach Anspruch 9, wobei der Wirtsorganismus das erste und das zweite Polypeptid in einem Verhältnis von 1 : 1 exprimieren kann.

12. Verfahren nach einem der Ansprüche 1 bis 11, das weiter eine nichtenzymatische Synthese von Forskolin aus (13R)-Manoyloxid umfasst.

13. (13R)-Manoyloxid, das durch das Verfahren nach einem der Ansprüche 1 bis 12 erhalten wurde, wobei das (13R)-Manoyloxid mehr als 90 % enantiomerenrein ist.

14. Isoliertes Diterpensynthase(diTPS)-Polypeptid der SEQ.-ID Nr. 1, 3, 4 oder 5 oder eine biologisch aktive Variante davon, die mindestens 90 % Sequenzidentität mit der Aminosäuresequenz, die in SEQ.-ID Nr. 1, 3, 4 oder 5 dargelegt ist, aufweist;
wobei die biologische Aktivität Diterpensynthase-Aktivität ist.

15. Polypeptid nach Anspruch 14, wobei das Polypeptid eine Klasse-I-diTPS-Aktivität aufweist und eine Sequenz aufweist, die mindestens 90 % identisch mit der Aminosäuresequenz ist, die in SEQ.-ID Nr. 3, 4 oder 5 dargelegt ist.

16. Polypeptid nach Anspruch 15, wobei das Polypeptid die Abspaltung einer Diphosphatgruppe von LPP und zusätzliche Cyclisierungs- oder Umlagerungsreaktionen an dem entstehenden Carbokation unter Erhalt von (13R)-Manoyloxid katalysieren kann.

17. Polypeptid nach Anspruch 14, wobei das Polypeptid eine Klasse-II-diTPS-Aktivität aufweist und eine Sequenz aufweist, die mindestens 90 % identisch mit der Aminosäuresequenz ist, die in SEQ.-ID Nr. 1 dargelegt ist, wobei das Polypeptid die Cycloisomerisierung von GGPP zu LPP katalysieren kann.

18. Polypeptid nach einem der Ansprüche 14 bis 17, das weiter ein Plastid-Zielsignal umfasst.

19. Polynukleotid, das ein Polypeptid nach einem der Ansprüche 14 bis 18 kodiert.

20. Polynukleotid nach Anspruch 19, wobei das Polynukleotid eine Sequenz aufweist, die mindestens 85 % identisch mit der Nukleotidsequenz ist, die in SEQ.-ID Nr. 6, 8, 9 oder 10 dargelegt ist.

21. Polynukleotid nach einem der Ansprüche 19 oder 20, das weiter eine Sequenz umfasst, die ein Plastid-Zielsignal kodiert.

22. Polynukleotid nach einem der Ansprüche 19 bis 21, wobei das Polynukleotid ist für die Expression in einer Wirtszelle Codon-optimiert, die eine Bakterienzelle, eine Hefezelle, eine Pilzzelle, eine Pflanzenzelle oder eine Insektenzelle ist.

23. Vektor, der ein Polynukleotid nach einem der Ansprüche 19 bis 22 umfasst.

24. Wirtszelle, die das Polynukleotid nach einem der Ansprüche 19 bis 22 und/oder den Vektor nach Anspruch 23 umfasst.

25. Zelle nach Anspruch 24, wobei die Zelle Folgendes exprimiert:
(i) ein erstes Polypeptid der SEQ.-ID Nr. 1 oder eine biologisch aktive Variante davon, die mindestens 80 % Sequenzidentität mit der Aminosäuresequenz, die in SEQ.-ID Nr. 1 dargelegt ist, aufweist; und
(b) ein zweites Polypeptid der SEQ.-ID Nr. 3, 4 oder 5 oder eine biologisch aktive Variante davon, die mindestens 80 % Sequenzidentität mit der Aminosäuresequenz, die in SEQ.-ID Nr. 3, 4 oder 5 dargelegt ist, aufweist,
wobei die Zelle eine Bakterienzelle, eine Hefezelle, eine Pilzzelle, eine Pflanzenzelle oder eine Insektenzelle ist.

26. Zelle nach Anspruch 25, wobei die Zelle eine *Nicotiana-benthamiana*-Zelle ist, die mit mindestens einem viralen Vektor für die transiente Expression des ersten und des zweiten Polypeptids transfiziert wurde.

27. Zelle nach einem der Ansprüche 24 bis 26, wobei die Zelle weiter GGPP natürlich erzeugen kann oder weiter zur Erzeugung von GGPP über den plastidären Methylerythritol-4-phosphat(MEP)-Weg konstruiert wurde.

28. Polypeptid nach Anspruch 14, wobei das Polypeptid weiter eine funktionsfähige Klasse-I-DDxxD-Domäne umfasst.

## Revendications

1. Méthode de préparation d'oxyde de (13R)-manoyle, comprenant :
(i) la fourniture de diphosphate de géranylgéranyle (GGPP) ;
(ii) la mise en contact du GGPP avec un premier polypeptide ayant au moins 75% d'identité de séquence avec la séquence d'acides aminés exposée dans SEQ ID n° 1 ou SEQ ID n° 2, pour obtenir ainsi le diphosphate de labd-13-ène-8,15-diol (LPP) ;
(iii) la mise en contact du LPP avec un deuxième polypeptide ayant au moins 75% d'identité de séquence avec la séquence d'acides aminés exposée dans SEQ ID n° 3, SEQ ID n° 4 ou SEQ ID n° 5 ;
afin de produire ainsi l'oxyde de (13R)-manoyle.

2. Méthode selon la revendication 1, où l'oxyde de (13R)-manoyle est énantiomériquement pur.

3. Méthode selon la revendication 1 ou 2, où le premier polypeptide et le deuxième polypeptide sont présents selon un rapport compris entre 2:1 et 1:2.

4. Méthode selon l'une quelconque des revendications 1 à 3, comprenant en outre une étape de récupération de l'oxyde de (13R)-manoyle.

5. Méthode selon l'une quelconque des revendications 1 à 4, où la méthode est mise en oeuvre *in vivo.*

6. Méthode selon l'une quelconque des revendications 1 à 5, où le premier polypeptide et le deuxième polypeptide sont exprimés de manière hétérologue dans un organisme hôte qui est des bactéries, des levures, des champignons, des plantes ou des insectes.

7. Méthode selon l'une quelconque des revendications 1 à 6, où l'organisme hôte est *Escherichia coli*, *Saccharomyces cerevisiae, Schizosaccharomyces pombe, Nicotiana benthamiana* ou *Physcomitrella patens.*

8. Méthode selon l'une quelconque des revendications 1 à 7, où le premier polypeptide et le deuxième polypeptide sont purifiés après une expression hétérologue.

9. Méthode selon l'une quelconque des revendications 1 à 8, où le GGPP est fourni dans une composition ou est produit par l'organisme hôte.

10. Méthode selon l'une quelconque des revendications 1 à 9, où
(a) le premier polypeptide possède au moins 75% d'identité de séquence avec la séquence d'acides aminés exposée dans SEQ ID n° 1 ; et
(b) le deuxième polypeptide possède au moins 75% d'identité de séquence avec la séquence d'acides aminés exposée dans SEQ ID n° 3.

11. Méthode selon la revendication 9, où l'organisme hôte est capable d'exprimer le premier et le deuxième polypeptide selon un rapport de 1:1.

12. Méthode selon l'une quelconque des revendications 1 à 11, comprenant en outre une synthèse non enzymatique de forskoline à partir de l'oxyde de (13R)-manoyle.

13. Oxyde de (13R)-manoyle obtenu par la méthode selon l'une quelconque des revendications 1 à 12, où l'oxyde de (13R)-manoyle est énantiomériquement pur à plus de 90%.

14. Polypeptide de diterpène synthase (diTPS) isolé de SEQ ID n° 1, 3, 4 ou 5 ou un variant biologiquement actif de celui-ci ayant au moins 90% d'identité de séquence avec la séquence d'acides aminés exposée dans SEQ ID n° 1, 3, 4 ou 5, où l'activité biologique est une activité de diterpène synthase.

15. Polypeptide selon la revendication 14, où le polypeptide possède une activité de diTPS de classe I, et possède une séquence ayant au moins 90% d'identité avec la séquence d'acides aminés exposée dans SEQ ID n° 3, 4 ou 5.

16. Polypeptide selon la revendication 15, où le polypeptide est capable de catalyser le clivage d'un groupement diphosphate de LPP et des réactions supplémentaires de cyclisation ou de réarrangement sur le carbocation résultant, conduisant à l'oxyde de (13R)-manoyle.

17. Polypeptide selon la revendication 14, où le polypeptide possède une activité de diTPS de classe II, et possède une séquence ayant au moins 90% d'identité avec la séquence d'acides aminés exposée dans SEQ ID n° 1, où le polypeptide est capable de catalyser la cycloisomérisation de GGPP en LPP.

18. Polypeptide selon l'une quelconque des revendications 14 à 17, comprenant en outre un signal de ciblage plastidial.

19. Polynucléotide codant pour un polypeptide selon l'une quelconque des revendications 14 à 18.

20. Polynucléotide selon la revendication 19, où le polynucléotide possède une séquence ayant au moins 85% d'identité avec la séquence nucléotidique exposée dans SEQ ID n° 6, 8, 9 ou 10.

21. Polynucléotide selon l'une quelconque des revendications 19 ou 20, comprenant en outre une séquence codant pour un signal de ciblage plastidial.

22. Polynucléotide selon l'une quelconque des revendications 19 à 21, où le polynucléotide est à codons optimisés pour une expression dans une cellule hôte qui est une cellule bactérienne, une cellule de levure, une cellule fongique, une cellule végétale ou une cellule d'insecte.

23. Vecteur comprenant un polynucléotide selon l'une quelconque des revendications 19 à 22.

24. Cellule hôte comprenant le polynucléotide selon l'une quelconque des revendications 19 à 22 et/ou le vecteur selon la revendication 23.

25. Cellule selon la revendication 24, où la cellule exprime :
(i) un premier polypeptide de SEQ ID n° 1 ou un variant biologiquement actif de celui-ci ayant au moins 80% d'identité de séquence avec la séquence d'acides aminés exposée dans SEQ ID n° 1 ; et
(ii) un deuxième polypeptide de SEQ ID n° 3, 4 ou 5 ou un variant biologiquement actif de celui-ci ayant au moins 80% d'identité de séquence avec la séquence d'acides aminés exposée dans SEQ ID n° 3, 4 ou 5,
où la cellule est une cellule bactérienne, une cellule de levure, une cellule fongique, une cellule végétale ou une cellule d'insecte.

26. Cellule selon la revendication 25, où la cellule est une cellule de *Nicotiana benthamiana* transfectée par au moins un vecteur viral pour l'expression transitoire du premier et du deuxième polypeptide.

27. Cellule selon l'une quelconque des revendications 24 à 26, où la cellule est en outre naturellement capable de produire ou modifiée davantage afin de produire du GGPP via la voie plastidiale du méthylérythritol-4-phosphate (MEP).

28. Polypeptide selon la revendication 14, où le polypeptide comprend en outre un domaine DDxxD de classe I fonctionnel.


### SEQUENCE LISTING

<110> Technical University of Denmark
<120> Stereo-specific biosynthesis of (13R) manoyl oxide
<130> P3406EP00
<160> 63
<170> PatentIn version 3.5
<210> 1
   <211> 773
   <212> PRT
   <213> Unknown
<220>
   <223> TPS2 from Coleus forskohlii
<220>
   <221> CfTPS2
   <222> (1)..(773)
   <223> Coleus forskohlii TPS2
<400> 1
   Met Lys Met Leu Met Ile Lys Ser Gln Phe Arg Val His Ser Ile Val
   1               5                   10                  15
   Ser Ala Trp Ala Asn Asn Ser Asn Lys Arg Gln Ser Leu Gly His Gln
               20                  25                  30
   Ile Arg Arg Lys Gln Arg Ser Gln Val Thr Glu Cys Arg Val Ala Ser
           35                  40                  45
   Leu Asp Ala Leu Asn Gly Ile Gln Lys Val Gly Pro Ala Thr Ile Gly
       50                  55                  60
   Thr Pro Glu Glu Glu Asn Lys Lys Ile Glu Asp Ser Ile Glu Tyr Val
   65                  70                  75                  80
   Lys Glu Leu Leu Lys Thr Met Gly Asp Gly Arg Ile Ser Val Ser Pro
                   85                  90                  95
   Tyr Asp Thr Ala Ile Val Ala Leu Ile Lys Asp Leu Glu Gly Gly Asp
               100                 105                 110
   Gly Pro Glu Phe Pro Ser Cys Leu Glu Trp Ile Ala Gln Asn Gln Leu
           115                 120                 125
   Ala Asp Gly Ser Trp Gly Asp His Phe Phe Cys Ile Tyr Asp Arg Val
       130                 135                 140
   Val Asn Thr Ala Ala Cys Val Val Ala Leu Lys Ser Trp Asn Val His
   145 150 155 160
   Ala Asp Lys Ile Glu Lys Gly Ala Val Tyr Leu Lys Glu Asn Val His
   165 170 175
   Lys Leu Lys Asp Gly Lys Ile Glu His Met Pro Ala Gly Phe Glu Phe
   180 185 190
   Val Val Pro Ala Thr Leu Glu Arg Ala Lys Ala Leu Gly Ile Lys Gly
   195 200 205
   Leu Pro Tyr Asp Asp Pro Phe Ile Arg Glu Ile Tyr Ser Ala Lys Gln
   210 215 220
   Thr Arg Leu Thr Lys Ile Pro Lys Gly Met Ile Tyr Glu Ser Pro Thr
   225 230 235 240
   Ser Leu Leu Tyr Ser Leu Asp Gly Leu Glu Gly Leu Glu Trp Asp Lys
   245 250 255
   Ile Leu Lys Leu Gln Ser Ala Asp Gly Ser Phe Ile Thr Ser Val Ser
   260 265 270
   Ser Thr Ala Phe Val Phe Met His Thr Asn Asp Leu Lys Cys His Ala
   275 280 285
   Phe Ile Lys Asn Ala Leu Thr Asn Cys Asn Gly Gly Val Pro His Thr
   290 295 300
   Tyr Pro Val Asp Ile Phe Ala Arg Leu Trp Ala Val Asp Arg Leu Gln
   305 310 315 320
   Arg Leu Gly Ile Ser Arg Phe Phe Glu Pro Glu Ile Lys Tyr Leu Met
   325 330 335
   Asp His Ile Asn Asn Val Trp Arg Glu Lys Gly Val Phe Ser Ser Arg
   340 345 350
   His Ser Gln Phe Ala Asp Ile Asp Asp Thr Ser Met Gly Ile Arg Leu
   355 360 365
   Leu Lys Met His Gly Tyr Asn Val Asn Pro Asn Ala Leu Glu His Phe
   370 375 380
   Lys Gln Lys Asp Gly Lys Phe Thr Cys Tyr Ala Asp Gln His Ile Glu
   385 390 395 400
   Ser Pro Ser Pro Met Tyr Asn Leu Tyr Arg Ala Ala Gln Leu Arg Phe
                   405                 410                 415
   Pro Gly Glu Glu Ile Leu Gln Gln Ala Leu Gln Phe Ala Tyr Asn Phe
               420                 425                 430
   Leu His Glu Asn Leu Ala Ser Asn His Phe Gln Glu Lys Trp Val Ile
           435                 440                 445
   Ser Asp His Leu Ile Asp Glu Val Arg Ile Gly Leu Lys Met Pro Trp
       450                 455                 460
   Tyr Ala Thr Leu Pro Arg Val Glu Ala Ser Tyr Tyr Leu Gln His Tyr
   465                 470                 475                 480
   Gly Gly Ser Ser Asp Val Trp Ile Gly Lys Thr Leu Tyr Arg Met Pro
                   485                 490                 495
   Glu Ile Ser Asn Asp Thr Tyr Lys Ile Leu Ala Gln Leu Asp Phe Asn
               500                 505                 510
   Lys Cys Gln Ala Gln His Gln Leu Glu Trp Met Ser Met Lys Glu Trp
           515                 520                 525
   Tyr Gln Ser Asn Asn Val Lys Glu Phe Gly Ile Ser Lys Lys Glu Leu
       530                 535                 540
   Leu Leu Ala Tyr Phe Leu Ala Ala Ala Thr Met Phe Glu Pro Glu Arg
   545                 550                 555                 560
   Thr Gln Glu Arg Ile Met Trp Ala Lys Thr Gln Val Val Ser Arg Met
                   565                 570                 575
   Ile Thr Ser Phe Leu Asn Lys Glu Asn Thr Met Ser Phe Asp Leu Lys
               580                 585                 590
   Ile Ala Leu Leu Thr Gln Pro Gln His Gln Ile Asn Gly Ser Glu Met
           595                 600                 605
   Lys Asn Gly Leu Ala Gln Thr Leu Pro Ala Ala Phe Arg Gln Leu Leu
       610                 615                 620
   Lys Glu Phe Asp Lys Tyr Thr Arg His Gln Leu Arg Asn Thr Trp Asn
   625                 630                 635                 640
   Lys Trp Leu Met Lys Leu Lys Gln Gly Asp Asp Asn Gly Gly Ala Asp
   645 650 655
   Ala Glu Leu Leu Ala Asn Thr Leu Asn Ile Cys Ala Gly His Asn Glu
   660 665 670
   Asp Ile Leu Ser His Tyr Glu Tyr Thr Ala Leu Ser Ser Leu Thr Asn
   675 680 685
   Lys Ile Cys Gln Arg Leu Ser Gln Ile Gln Asp Lys Lys Met Leu Glu
   690 695 700
   Ile Glu Glu Gly Ser Ile Lys Asp Lys Glu Met Glu Leu Glu Ile Gln
   705 710 715 720
   Thr Leu Val Lys Leu Val Leu Gln Glu Thr Ser Gly Gly Ile Asp Arg
   725 730 735
   Asn Ile Lys Gln Thr Phe Leu Ser Val Phe Lys Thr Phe Tyr Tyr Arg
   740 745 750
   Ala Tyr His Asp Ala Lys Thr Ile Asp Ala His Ile Phe Gln Val Leu
   755 760 765
   Phe Glu Pro Val Val
   770
<210> 2
   <211> 785
   <212> PRT
   <213> Salvia sclarea
<220>
   <221> SsLPPS
   <222> (1)..(785)
<400> 2
   Met Thr Ser Val Asn Leu Ser Arg Ala Pro Ala Ala Ile Thr Arg Arg
   1 5 10 15
   Arg Leu Gln Leu Gln Pro Glu Phe His Ala Glu Cys Ser Trp Leu Lys
   20 25 30
   Ser Ser Ser Lys His Ala Pro Leu Thr Leu Ser Cys Gln Ile Arg Pro
   35 40 45
   Lys Gln Leu Ser Gln Ile Ala Glu Leu Arg Val Thr Ser Leu Asp Ala
   50 55 60
   Ser Gln Ala Ser Glu Lys Asp Ile Ser Leu Val Gln Thr Pro His Lys
   65                  70                  75                  80
   Val Glu Val Asn Glu Lys Ile Glu Glu Ser Ile Glu Tyr Val Gln Asn
                   85                  90                  95
   Leu Leu Met Thr Ser Gly Asp Gly Arg Ile Ser Val Ser Pro Tyr Asp
               100                 105                 110
   Thr Ala Val Ile Ala Leu Ile Lys Asp Leu Lys Gly Arg Asp Ala Pro
           115                 120                 125
   Gln Phe Pro Ser Cys Leu Glu Trp Ile Ala His His Gln Leu Ala Asp
       130                 135                 140
   Gly Ser Trp Gly Asp Glu Phe Phe Cys Ile Tyr Asp Arg Ile Leu Asn
   145                 150                 155                 160
   Thr Leu Ala Cys Val Val Ala Leu Lys Ser Trp Asn Leu His Ser Asp
                   165                 170                 175
   Ile Ile Glu Lys Gly Val Thr Tyr Ile Lys Glu Asn Val His Lys Leu
               180                 185                 190
   Lys Gly Ala Asn Val Glu His Arg Thr Ala Gly Phe Glu Leu Val Val
           195                 200                 205
   Pro Thr Phe Met Gln Met Ala Thr Asp Leu Gly Ile Gln Asp Leu Pro
       210                 215                 220
   Tyr Asp His Pro Leu Ile Lys Glu Ile Ala Asp Thr Lys Gln Gln Arg
   225                 230                 235                 240
   Leu Lys Glu Ile Pro Lys Asp Leu Val Tyr Gln Met Pro Thr Asn Leu
                   245                 250                 255
   Leu Tyr Ser Leu Glu Gly Leu Gly Asp Leu Glu Trp Glu Arg Leu Leu
               260                 265                 270
   Lys Leu Gln Ser Gly Asn Gly Ser Phe Leu Thr Ser Pro Ser Ser Thr
           275                 280                 285
   Ala Ala Val Leu Met His Thr Lys Asp Glu Lys Cys Leu Lys Tyr Ile
       290                 295                 300
   Glu Asn Ala Leu Lys Asn Cys Asp Gly Gly Ala Pro His Thr Tyr Pro
   305                 310                 315                 320
   Val Asp Ile Phe Ser Arg Leu Trp Ala Ile Asp Arg Leu Gln Arg Leu
                   325                 330                 335
   Gly Ile Ser Arg Phe Phe Gln His Glu Ile Lys Tyr Phe Leu Asp His
               340                 345                 350
   Ile Glu Ser Val Trp Glu Glu Thr Gly Val Phe Ser Gly Arg Tyr Thr
           355                 360                 365
   Lys Phe Ser Asp Ile Asp Asp Thr Ser Met Gly Val Arg Leu Leu Lys
       370                 375                 380
   Met His Gly Tyr Asp Val Asp Pro Asn Val Leu Lys His Phe Lys Gln
   385                 390                 395                 400
   Gln Asp Gly Lys Phe Ser Cys Tyr Ile Gly Gln Ser Val Glu Ser Ala
                   405                 410                 415
   Ser Pro Met Tyr Asn Leu Tyr Arg Ala Ala Gln Leu Arg Phe Pro Gly
               420                 425                 430
   Glu Glu Val Leu Glu Glu Ala Thr Lys Phe Ala Phe Asn Phe Leu Gln
           435                 440                 445
   Glu Met Leu Val Lys Asp Arg Leu Gln Glu Arg Trp Val Ile Ser Asp
       450                 455                 460
   His Leu Phe Asp Glu Ile Lys Leu Gly Leu Lys Met Pro Trp Tyr Ala
   465                 470                 475                 480
   Thr Leu Pro Arg Val Glu Ala Ala Tyr Tyr Leu Asp His Tyr Ala Gly
                   485                 490                 495
   Ser Gly Asp Val Trp Ile Gly Lys Ser Phe Tyr Arg Met Pro Glu Ile
               500                 505                 510
   Ser Asn Asp Thr Tyr Lys Glu Leu Ala Ile Leu Asp Phe Asn Arg Cys
           515                 520                 525
   Gln Thr Gln His Gln Leu Glu Trp Ile His Met Gln Glu Trp Tyr Asp
       530                 535                 540
   Arg Cys Ser Leu Ser Glu Phe Gly Ile Ser Lys Arg Glu Leu Leu Arg
   545                 550                 555                 560
   Ser Tyr Phe Leu Ala Ala Ala Thr Ile Phe Glu Pro Glu Arg Thr Gln
                   565                 570                 575
   Glu Arg Leu Leu Trp Ala Lys Thr Arg Ile Leu Ser Lys Met Ile Thr
               580                 585                 590
   Ser Phe Val Asn Ile Ser Gly Thr Thr Leu Ser Leu Asp Tyr Asn Phe
           595                 600                 605
   Asn Gly Leu Asp Glu Ile Ile Ser Ser Ala Asn Glu Asp Gln Gly Leu
       610                 615                 620
   Ala Gly Thr Leu Leu Ala Thr Phe His Gln Leu Leu Asp Gly Phe Asp
   625                 630                 635                 640
   Ile Tyr Thr Leu His Gln Leu Lys His Val Trp Ser Gln Trp Phe Met
                   645                 650                 655
   Lys Val Gln Gln Gly Glu Gly Ser Gly Gly Glu Asp Ala Val Leu Leu
               660                 665                 670
   Ala Asn Thr Leu Asn Ile Cys Ala Gly Leu Asn Glu Asp Val Leu Ser
           675                 680                 685
   Asn Asn Glu Tyr Thr Ala Leu Ser Thr Leu Thr Asn Lys Ile Cys Asn
       690                 695                 700
   Arg Leu Ala Gln Ile Gln Asp Asn Lys Ile Leu Gln Val Val Asp Gly
   705                 710                 715                 720
   Ser Ile Lys Asp Lys Glu Leu Glu Gln Asp Met Gln Ala Leu Val Lys
                   725                 730                 735
   Leu Val Leu Gln Glu Asn Gly Gly Ala Val Asp Arg Asn Ile Arg His
               740                 745                 750
   Thr Phe Leu Ser Val Ser Lys Thr Phe Tyr Tyr Asp Ala Tyr His Asp
           755                 760                 765
   Asp Glu Thr Thr Asp Leu His Ile Phe Lys Val Leu Phe Arg Pro Val
       770                 775                 780
   Val
   785
<210> 3
   <211> 587
   <212> PRT
   <213> Unknown
<220>
   <223> CfTPS4 from Coleus forskohlii
<220>
   <221> CfTPS4
   <222> (1)..(587)
<400> 3
   Met Ser Ile Thr Ile Asn Leu Arg Val Ile Ala Phe Pro Gly His Gly
   1               5                   10                  15
   Val Gln Ser Arg Gln Gly Ile Phe Ala Val Met Glu Phe Pro Arg Asn
               20                  25                  30
   Lys Asn Thr Phe Lys Ser Ser Phe Ala Val Lys Cys Ser Leu Ser Thr
           35                  40                  45
   Pro Thr Asp Leu Met Gly Lys Ile Lys Glu Lys Leu Arg Glu Lys Val
       50                  55                  60
   Asp Asn Ser Gly Ala Ala Met Ala Thr Asp Ser Ala Asp Met Pro Thr
   65                  70                  75                  80
   Asn Leu Cys Ile Val Asp Ser Leu Gln Arg Leu Gly Val Glu Lys Tyr
                   85                  90                  95
   Phe Gln Ser Glu Ile Asp Thr Val Leu Asp Asp Ala Tyr Arg Leu Trp
               100                 105                 110
   Gln Leu Lys Gln Lys Asp Ile Phe Ser Asp Ile Thr Thr His Ala Met
           115                 120                 125
   Ala Phe Arg Leu Leu Arg Val Lys Gly Tyr Asp Val Ser Ser Glu Glu
       130                 135                 140
   Leu Ala Pro Tyr Ala Asp Gln Glu Gly Met Asn Leu Gln Thr Ile Asp
   145                 150                 155                 160
   Leu Ala Ala Val Ile Glu Leu Tyr Arg Ala Ala Gln Glu Arg Val Ala
                   165                 170                 175
   Glu Glu Asp Ser Thr Leu Glu Lys Leu Tyr Val Trp Thr Ser Thr Phe
               180                 185                 190
   Leu Lys Gln Gln Leu Leu Ala Gly Ala Ile Pro Asp Gln Lys Leu His
           195                 200                 205
   Lys Gln Val Glu Tyr Tyr Leu Lys Asn Tyr His Gly Ile Leu Asp Arg
   210 215 220
   Met Gly Val Arg Lys Gly Leu Asp Leu Tyr Asp Ala Gly Tyr Tyr Lys
   225 230 235 240
   Ala Leu Lys Ala Ala Asp Arg Leu Val Asp Leu Cys Asn Glu Asp Leu
   245 250 255
   Leu Ala Phe Ala Arg Gln Asp Phe Asn Ile Asn Gln Ala Gln His Arg
   260 265 270
   Lys Glu Leu Glu Gln Leu Gln Arg Trp Tyr Ala Asp Cys Arg Leu Asp
   275 280 285
   Lys Leu Glu Phe Gly Arg Asp Val Val Arg Val Ser Asn Phe Leu Thr
   290 295 300
   Ser Ala Ile Leu Gly Asp Pro Glu Leu Ser Glu Val Arg Leu Val Phe
   305 310 315 320
   Ala Lys His Ile Val Leu Val Thr Arg Ile Asp Asp Phe Phe Asp His
   325 330 335
   Gly Gly Pro Arg Glu Glu Ser His Lys Ile Leu Glu Leu Ile Lys Glu
   340 345 350
   Trp Lys Glu Lys Pro Ala Gly Glu Tyr Val Ser Lys Glu Val Glu Ile
   355 360 365
   Leu Tyr Thr Ala Val Tyr Asn Thr Val Asn Glu Leu Ala Glu Arg Ala
   370 375 380
   Asn Val Glu Gln Gly Arg Asn Val Glu Pro Phe Leu Arg Thr Leu Trp
   385 390 395 400
   Val Gln Ile Leu Ser Ile Phe Lys Ile Glu Leu Asp Thr Trp Ser Asp
   405 410 415
   Asp Thr Ala Leu Thr Leu Asp Asp Tyr Leu Asn Asn Ser Trp Val Ser
   420 425 430
   Ile Gly Cys Arg Ile Cys Ile Leu Met Ser Met Gln Phe Ile Gly Met
   435 440 445
   Lys Leu Pro Glu Glu Met Leu Leu Ser Glu Glu Cys Val Asp Leu Cys
   450 455 460
   Arg His Val Ser Met Val Asp Arg Leu Leu Asn Asp Val Gln Thr Phe
   465 470 475 480
   Glu Lys Glu Arg Lys Glu Asn Thr Gly Asn Ala Val Ser Leu Leu Leu
   485 490 495
   Ala Ala His Lys Gly Glu Arg Ala Phe Ser Glu Glu Glu Ala Ile Ala
   500 505 510
   Lys Ala Lys Tyr Leu Ala Asp Cys Asn Arg Arg Ser Leu Met Gln Ile
   515 520 525
   Val Tyr Lys Thr Gly Thr Ile Phe Pro Arg Lys Cys Lys Asp Met Phe
   530 535 540
   Leu Lys Val Cys Arg Ile Gly Cys Tyr Leu Tyr Ala Ser Gly Asp Glu
   545 550 555 560
   Phe Thr Ser Pro Gln Gln Met Met Glu Asp Met Lys Ser Leu Val Tyr
   565 570 575
   Glu Pro Leu Gln Ile His Pro Pro Pro Ala Asn
   580 585
<210> 4
   <211> 598
   <212> PRT
   <213> Unknown
<220>
   <223> TPS3 from Coleus forskohlii
<220>
   <221> CfTPS3
   <222> (1)..(598)
<400> 4
   Met Ser Ser Leu Ala Gly Asn Leu Arg Val Ile Pro Phe Ser Gly Asn
   1 5 10 15
   Arg Val Gln Thr Arg Thr Gly Ile Leu Pro Val His Gln Thr Pro Met
   20 25 30
   Ile Thr Ser Lys Ser Ser Ala Ala Val Lys Cys Ser Leu Thr Thr Pro
   35 40 45
   Thr Asp Leu Met Gly Lys Ile Lys Glu Val Phe Asn Arg Glu Val Asp
   50 55 60
   Thr Ser Pro Ala Ala Met Thr Thr His Ser Thr Asp Ile Pro Ser Asn
   65                  70                  75                  80
   Leu Cys Ile Ile Asp Thr Leu Gln Arg Leu Gly Ile Asp Gln Tyr Phe
                   85                  90                  95
   Gln Ser Glu Ile Asp Ala Val Leu His Asp Thr Tyr Arg Leu Trp Gln
               100                 105                 110
   Leu Lys Lys Lys Asp Ile Phe Ser Asp Ile Thr Thr His Ala Met Ala
           115                 120                 125
   Phe Arg Leu Leu Arg Val Lys Gly Tyr Glu Val Ala Ser Asp Glu Leu
       130                 135                 140
   Ala Pro Tyr Ala Asp Gln Glu Arg Ile Asn Leu Gln Thr Ile Asp Val
   145                 150                 155                 160
   Pro Thr Val Val Glu Leu Tyr Arg Ala Ala Gln Glu Arg Leu Thr Glu
                   165                 170                 175
   Glu Asp Ser Thr Leu Glu Lys Leu Tyr Val Trp Thr Ser Ala Phe Leu
               180                 185                 190
   Lys Gln Gln Leu Leu Thr Asp Ala Ile Pro Asp Lys Lys Leu His Lys
           195                 200                 205
   Gln Val Glu Tyr Tyr Leu Lys Asn Tyr His Gly Ile Leu Asp Arg Met
       210                 215                 220
   Gly Val Arg Arg Asn Leu Asp Leu Tyr Asp Ile Ser His Tyr Lys Ser
   225                 230                 235                 240
   Leu Lys Ala Ala His Arg Phe Tyr Asn Leu Ser Asn Glu Asp Ile Leu
                   245                 250                 255
   Ala Phe Ala Arg Gln Asp Phe Asn Ile Ser Gln Ala Gln His Gln Lys
               260                 265                 270
   Glu Leu Gln Gln Leu Gln Arg Trp Tyr Ala Asp Cys Arg Leu Asp Thr
           275                 280                 285
   Leu Lys Phe Gly Arg Asp Val Val Arg Ile Gly Asn Phe Leu Thr Ser
       290                 295                 300
   Ala Met Ile Gly Asp Pro Glu Leu Ser Asp Leu Arg Leu Ala Phe Ala
   305                 310                 315                 320
   Lys His Ile Val Leu Val Thr Arg Ile Asp Asp Phe Phe Asp His Gly
                   325                 330                 335
   Gly Pro Lys Glu Glu Ser Tyr Glu Ile Leu Glu Leu Val Lys Glu Trp
               340                 345                 350
   Lys Glu Lys Pro Ala Gly Glu Tyr Val Ser Glu Glu Val Glu Ile Leu
           355                 360                 365
   Phe Thr Ala Val Tyr Asn Thr Val Asn Glu Leu Ala Glu Met Ala His
       370                 375                 380
   Ile Glu Gln Gly Arg Ser Val Lys Asp Leu Leu Val Lys Leu Trp Val
   385                 390                 395                 400
   Glu Ile Leu Ser Val Phe Arg Ile Glu Leu Asp Thr Trp Thr Asn Asp
                   405                 410                 415
   Thr Ala Leu Thr Leu Glu Glu Tyr Leu Ser Gln Ser Trp Val Ser Ile
               420                 425                 430
   Gly Cys Arg Ile Cys Ile Leu Ile Ser Met Gln Phe Gln Gly Val Lys
           435                 440                 445
   Leu Ser Asp Glu Met Leu Gln Ser Glu Glu Cys Thr Asp Leu Cys Arg
       450                 455                 460
   Tyr Val Ser Met Val Asp Arg Leu Leu Asn Asp Val Gln Thr Phe Glu
   465                 470                 475                 480
   Lys Glu Arg Lys Glu Asn Thr Gly Asn Ser Val Ser Leu Leu Gln Ala
                   485                 490                 495
   Ala His Lys Asp Glu Arg Val Ile Asn Glu Glu Glu Ala Cys Ile Lys
               500                 505                 510
   Val Lys Glu Leu Ala Glu Tyr Asn Arg Arg Lys Leu Met Gln Ile Val
           515                 520                 525
   Tyr Lys Thr Gly Thr Ile Phe Pro Arg Lys Cys Lys Asp Leu Phe Leu
       530                 535                 540
   Lys Ala Cys Arg Ile Gly Cys Tyr Leu Tyr Ser Ser Gly Asp Glu Phe
   545                 550                 555                 560
   Thr Ser Pro Gln Gln Met Met Glu Asp Met Lys Ser Leu Val Tyr Glu
                   565                 570                 575
   Pro Leu Pro Ile Ser Pro Pro Glu Ala Asn Asn Ala Ser Gly Glu Lys
   580 585 590
   Met Ser Cys Val Ser Asn
   595
<210> 5
   <211> 792
   <212> PRT
   <213> Euphorbia peplus
<220>
   <221> EpTPS8
   <222> (1)..(792)
<400> 5
   Met Gln Val Ser Leu Ser Leu Thr Thr Gly Ser Glu Pro Cys Ile Thr
   1 5 10 15
   Arg Ile His Ala Pro Ser Asp Ala Pro Leu Lys Gln Arg Asn Asn Glu
   20 25 30
   Arg Glu Lys Gly Thr Leu Glu Leu Asn Gly Lys Val Ser Leu Lys Lys
   35 40 45
   Met Gly Glu Met Leu Arg Thr Ile Glu Asn Val Pro Ile Val Gly Ser
   50 55 60
   Thr Ser Ser Tyr Asp Thr Ala Trp Val Gly Met Val Pro Cys Ser Ser
   65 70 75 80
   Asn Ser Ser Lys Pro Leu Phe Pro Glu Ser Leu Lys Trp Ile Met Glu
   85 90 95
   Asn Gln Asn Pro Glu Gly Asn Trp Ala Val Asp His Ala His His Pro
   100 105 110
   Leu Leu Leu Lys Asp Ser Leu Ser Ser Thr Leu Ala Cys Val Leu Ala
   115 120 125
   Leu His Lys Trp Asn Leu Ala Pro Gln Leu Val His Ser Gly Leu Asp
   130 135 140
   Phe Ile Gly Ser Asn Leu Trp Ala Ala Met Asp Phe Arg Gln Arg Ser
   145 150 155 160
   Pro Leu Gly Phe Asp Val Ile Phe Pro Gly Met Ile His Gln Ala Ile
   165 170 175
   Asp Leu Gly Ile Asn Leu Pro Phe Asn Asn Ser Ser Ile Glu Asn Met
   180 185 190
   Leu Thr Asn Pro Leu Leu Asp Ile Gln Ser Phe Glu Ala Gly Lys Thr
   195 200 205
   Ser His Ile Ala Tyr Phe Ala Glu Gly Leu Gly Ser Arg Leu Lys Asp
   210 215 220
   Trp Glu Gln Leu Leu Gln Tyr Gln Thr Ser Asn Gly Ser Leu Phe Asn
   225 230 235 240
   Ser Pro Ser Thr Thr Ala Ala Ala Ala Ile His Leu Arg Asp Glu Lys
   245 250 255
   Cys Leu Asn Tyr Leu His Ser Leu Thr Lys Gln Phe Asp Asn Gly Ala
   260 265 270
   Val Pro Thr Leu Tyr Pro Leu Asp Ala Arg Thr Arg Ile Ser Ile Ile
   275 280 285
   Asp Ser Leu Glu Lys Phe Gly Ile His Ser His Phe Ile Gln Glu Met
   290 295 300
   Thr Ile Leu Leu Asp Gln Ile Tyr Ser Phe Trp Lys Glu Gly Asn Glu
   305 310 315 320
   Glu Ile Phe Lys Asp Pro Gly Cys Cys Ala Thr Ala Phe Arg Leu Leu
   325 330 335
   Arg Lys His Gly Tyr Asp Val Ser Ser Asp Ser Leu Ala Glu Phe Glu
   340 345 350
   Lys Lys Glu Ile Phe Tyr His Ser Ser Ala Ala Ser Ala His Glu Ile
   355 360 365
   Asp Thr Lys Ser Ile Leu Glu Leu Phe Arg Ala Ser Gln Met Lys Ile
   370 375 380
   Leu Gln Asn Glu Pro Ile Leu Asp Arg Ile Tyr Asp Trp Thr Ser Ile
   385 390 395 400
   Phe Leu Arg Asp Gln Leu Val Lys Gly Leu Ile Glu Asn Lys Ser Leu
   405 410 415
   Tyr Glu Glu Val Asn Phe Ala Leu Gly His Pro Phe Ala Asn Leu Asp
               420                 425                 430
   Arg Leu Glu Ala Arg Ser Tyr Ile Asp Asn Tyr Asp Pro Tyr Asp Val
           435                 440                 445
   Pro Leu Leu Lys Thr Ser Tyr Arg Ser Ser Asn Ile Asp Asn Lys Asp
       450                 455                 460
   Leu Trp Thr Ile Ala Phe Gln Asp Phe Asn Lys Cys Gln Ala Leu His
   465                 470                 475                 480
   Arg Val Glu Leu Asp Tyr Leu Glu Lys Trp Val Lys Glu Tyr Lys Leu
                   485                 490                 495
   Asp Thr Leu Lys Trp Ala Arg Gln Lys Thr Glu Tyr Ala Leu Phe Thr
               500                 505                 510
   Ile Gly Ala Ile Leu Ser Glu Pro Glu Tyr Ala Asp Ala Arg Ile Ser
           515                 520                 525
   Trp Ser Gln Asn Thr Val Phe Val Thr Ile Val Asp Asp Phe Phe Asp
       530                 535                 540
   Tyr Gly Gly Ser Leu Asp Glu Cys Arg Asn Leu Ile Asn Leu Met His
   545                 550                 555                 560
   Lys Trp Asp Asp His Leu Thr Val Gly Phe Leu Ser Glu Lys Val Glu
                   565                 570                 575
   Ile Val Phe Tyr Ser Met Tyr Gly Thr Leu Asn Asp Leu Ala Ala Lys
               580                 585                 590
   Ala Glu Val Arg Gln Gly Arg Cys Val Arg Ser His Leu Val Asn Leu
           595                 600                 605
   Trp Ile Trp Val Met Glu Asn Met Leu Lys Glu Arg Glu Trp Ala Asp
       610                 615                 620
   Tyr Asn Leu Val Pro Thr Phe Tyr Glu Tyr Val Ala Ala Gly His Ile
   625                 630                 635                 640
   Thr Ile Gly Leu Gly Pro Val Leu Leu Ile Ala Leu Tyr Phe Met Gly
                   645                 650                 655
   Tyr Pro Leu Ser Glu Asp Val Val Gln Ser Gln Glu Tyr Lys Gly Val
               660                 665                 670
   Tyr Leu Asn Val Ser Ile Ile Ala Arg Leu Leu Asn Asp Arg Val Thr
           675                 680                 685
   Val Lys Arg Glu Ser Ala Gln Gly Lys Leu Asn Gly Val Ser Leu Phe
       690                 695                 700
   Val Glu His Gly Arg Gly Ala Val Asp Glu Glu Thr Ser Met Lys Glu
   705                 710                 715                 720
   Val Glu Arg Leu Val Glu Ser His Lys Arg Glu Leu Leu Arg Leu Ile
                   725                 730                 735
   Val Gln Lys Thr Glu Gly Ser Val Val Pro Gln Ser Cys Lys Asp Leu
               740                 745                 750
   Ala Trp Arg Val Ser Lys Val Leu His Leu Leu Tyr Met Asp Asp Asp
           755                 760                 765
   Gly Phe Thr Cys Pro Val Lys Met Leu Asn Ala Thr Asn Ala Ile Val
       770                 775                 780
   Asn Glu Pro Leu Leu Leu Thr Ser
   785                 790
<210> 6
   <211> 2322
   <212> DNA
   <213> Unknown
<220>
   <223> CfTPS2 from Coleus forskohlii
<220>
   <221> CfTPS2
   <222> (1)..(2322)
<400> 6
   atgaaaatgt tgatgatcaa aagtcaattt cgtgtacatt caatagtcag tgcatgggcg      60
   aacaacagca ataaaaggca gtcattgggt caccaaattc gtcgaaagca aagatcacaa     120
   gtaaccgagt gtcgagttgc aagtctggat gcgttgaatg gaattcaaaa agtcggccca     180
   gccaccattg ggactcctga agaggaaaat aaaaagattg aggattccat tgagtacgtg     240
   aaggagttgt tgaagacaat gggcgacggg cgaatcagcg tttccccgta cgacacagca     300
   atagttgccc tgattaagga cttggaagga ggtgatggac cagagtttcc atcttgtcta     360
   gagtggattg cacagaatca actggctgat ggttcttggg gggatcactt cttctgtatt     420
   tatgatcggg ttgttaatac agcagcttgt gtggtcgcct taaagtcgtg gaacgttcac     480
   gcagacaaga ttgagaaagg agcagtgtac ctgaaggaga atgtgcataa acttaaagat 540
   gggaagattg agcacatgcc cgcagggttt gaatttgtgg ttcctgccac tcttgaaaga 600
   gccaaagcct tggggatcaa aggtcttccc tatgatgatc ctttcatcag ggaaatttat 660
   agtgcaaaac aaacaagatt gaccaagata ccaaagggca tgatctacga atctccaact 720
   tctttattat atagtttaga cggtctggaa ggcttggagt gggacaagat actgaaactg 780
   cagtcggccg atggctcatt catcacctct gtgtcgtcta ctgccttcgt attcatgcac 840
   accaacgacc ttaaatgcca cgccttcatc aaaaatgccc tcaccaattg caacggggga 900
   gtaccccaca cgtatccagt ggatatcttc gcacgacttt gggcagtgga ccgactgcaa 960
   cgcctcggaa tatctcgatt ctttgagcct gagatcaaat atttaatgga tcacatcaat 1020
   aacgtgtgga gggagaaggg agttttcagt tcaaggcatt cacaatttgc ggatattgac 1080
   gacacatcca tgggcatcag gcttctgaaa atgcacggat acaatgtcaa cccaaatgca 1140
   cttgaacatt tcaaacagaa agatgggaag tttacatgct atgctgatca acatatcgag 1200
   tctccatccc ccatgtacaa tctctacagg gctgctcagc ttcgttttcc aggagaagaa 1260
   attcttcaac aagcccttca atttgcctat aattttctac atgaaaacct agccagcaat 1320
   cactttcaag aaaaatgggt catatccgac cacctaattg atgaggtaag gatcgggctg 1380
   aagatgccat ggtacgccac cctaccgcga gtggaggctt catactatct tcaacattat 1440
   ggtggatcca gcgacgtatg gattggcaaa actttataca gaatgccaga aatcagtaac 1500
   gacacataca aaatacttgc acaattggac ttcaacaaat gtcaagcaca acatcagttg 1560
   gaatggatgt ccatgaaaga gtggtatcaa agtaataatg ttaaagaatt tgggataagc 1620
   aagaaagaac ttcttcttgc ttactttttg gctgctgcaa ccatgtttga acccgaacgc 1680
   acacaagaga ggattatgtg ggcgaaaact caagtcgttt ctcggatgat cacatcattt 1740
   ctcaacaaag aaaacacaat gtcattcgac ctaaagattg cacttttaac ccaaccccaa 1800
   catcaaataa atggttctga gatgaagaat ggacttgctc aaactcttcc tgcagccttc 1860
   cgacaactac tcaaggaatt cgacaaatac acaagacatc aattgaggaa tacttggaac 1920
   aaatggttga tgaaactgaa gcaaggagat gacaatggcg gcgcagatgc agagctcctt 1980
   gcaaacacat taaacatatg tgctggacat aacgaagaca tattatcgca ctatgaatac 2040
   accgctctct cctccctcac aaacaaaata tgtcagcgtc taagtcaaat tcaagataaa 2100
   aagatgctgg aaattgagga ggggagcata aaagataagg agatggagct cgaaatacaa 2160
   acattggtga agttagtcct ccaagaaacc agtgggggta tcgatagaaa catcaagcaa 2220
   acatttttat cagtattcaa gacattttac tacagggcct accacgatgc taagactatc 2280
   gatgcccata ttttccaagt actatttgaa ccagtggtct ga 2322
<210> 7
   <211> 2358
   <212> DNA
   <213> Salvia sclarea
<220>
   <221> SsLPPS
   <222> (1)..(2358)
<400> 7
   atgacttctg taaatttgag cagagcacca gcagcgatta cccggcgcag gctgcagcta      60
   cagccggaat ttcatgccga gtgttcatgg ctgaaaagca gcagcaaaca cgcgcccttg     120
   accttgagtt gccaaatccg tcctaagcaa ctctcccaaa tagctgaatt gagagtaaca     180
   agcctggatg cgtcgcaagc gagtgaaaaa gacatttccc ttgttcaaac tccgcataag     240
   gttgaggtta atgaaaagat cgaggagtca atcgagtacg tccaaaatct gttgatgacg     300
   tcgggcgacg ggcgaataag cgtgtcaccc tatgacacgg cagtgatcgc cctgatcaag     360
   gacttgaaag ggcgcgacgc cccgcagttt ccgtcatgtc tcgagtggat cgcgcaccac     420
   caactggctg atggctcatg gggcgacgaa ttcttctgta tttatgatcg gattctaaat      480
   acattggcat gtgtcgtagc cttgaaatca tggaaccttc actctgatat tattgaaaaa     540
   ggagtgacgt acatcaagga gaatgtgcat aaacttaaag gtgcaaatgt tgagcacagg     600
   acagcggggt tcgaacttgt ggttcctact tttatgcaaa tggccacaga tttgggcatc     660
   caagatctgc cctatgatca tcccctcatc aaggagattg ctgacacaaa acaacaaaga     720
   ttgaaagaga tacccaagga tttggtttac caaatgccaa cgaatttact gtacagttta     780
   gaagggttag gagatttgga gtgggaaagg ctactgaaac tgcagtcggg caatggctcc     840
   ttcctcactt cgccgtcgtc caccgccgcc gtcttgatgc ataccaaaga tgaaaaatgt     900
   ttgaaataca tcgaaaacgc cctcaagaat tgcgacggag gagcaccaca tacttatcca     960
   gtcgatatct tctcaagact ttgggcaatc gataggctac aacgcctagg aatttctcgt    1020
   ttcttccagc acgagatcaa gtatttctta gatcacatcg aaagcgtttg ggaggagacc    1080
   ggagttttca gtggaagata tacgaaattt agcgatattg atgacacgtc catgggcgtt    1140
   aggcttctca aaatgcacgg atacgacgtc gatccaaatg tactaaaaca tttcaagcaa    1200
   caagatggta aattttcctg ctacattggt caatcggtcg agtctgcatc tccaatgtac    1260
   aatctttata gggctgctca actaagattt ccaggagaag aagttcttga agaagccact    1320
   aaatttgcct ttaacttctt gcaagaaatg ctagtcaaag atcgacttca agaaagatgg    1380
   gtgatatccg accacttatt tgatgagata aagctggggt tgaagatgcc atggtacgcc    1440
   actctacccc gagtcgaggc tgcatattat ctagaccatt atgctggttc tggtgatgta    1500
   tggattggca agagtttcta caggatgcca gaaatcagca atgatacata caaggagctt    1560
   gcgatattgg atttcaacag atgccaaaca caacatcagt tggagtggat ccacatgcag      1620
   gaatggtacg acagatgcag ccttagcgaa ttcgggataa gcaaaagaga gttgcttcgc      1680
   tcttactttc tggccgcagc aaccatattc gaaccggaga gaactcaaga gaggcttctg      1740
   tgggccaaaa ccagaattct ttctaagatg atcacttcat ttgtcaacat tagtggaaca      1800
   acactatctt tggactacaa tttcaatggc ctcgatgaaa taattagtag tgccaatgaa      1860
   gatcaaggac tggctgggac tctgctggca accttccatc aacttctaga cggattcgat      1920
   atatacactc tccatcaact caaacatgtt tggagccaat ggttcatgaa agtgcagcaa      1980
   ggagagggaa gcggcgggga agacgcggtg ctcctagcga acacgctcaa catctgcgcc      2040
   ggcctcaacg aagacgtgtt gtccaacaat gaatacacgg ctctgtccac cctcacaaat      2100
   aaaatctgca atcgcctcgc ccaaattcaa gacaataaga ttctccaagt tgtggatggg      2160
   agcataaagg ataaggagct agaacaggat atgcaggcgt tggtgaagtt agtgcttcaa      2220
   gaaaatggcg gcgccgtaga cagaaacatc agacacacgt ttttgtcggt ttccaagact      2280
   ttctactacg atgcctacca cgacgatgag acgaccgatc ttcatatctt caaagtactc      2340
   tttcgaccgg ttgtatga                                                    2358
<210> 8
   <211> 1764
   <212> DNA
   <213> Unknown
<220>
   <223> CfTPS4 from Coleus forskohlii
<220>
   <221> CfTPS4
   <222> (1)..(1764)
<400> 8
   atgtcaatca ccatcaacct tcgagttatc gctttccccg gccatggagt tcagagcagg 60
   caaggaatat ttgcagtcat ggaatttcca aggaacaaga acacctttaa atcatccttt 120
   gctgttaaat gcagcctctc tactccaaca gatttgatgg gaaagataaa agaaaagttg 180
   agcgagaagg ttgataattc tgtggcagcc atggctactg attctgccga tatgcccact 240
   aatctctgca tcgtcgactc cctccagagg ctgggagtcg aaaaatattt ccaatccgaa 300
   atcgacactg ttctcgatga tgcataccgg ttatggcagc tgaagcagaa agatatattt 360
   tcagacatta ctactcatgc aatggcgttt agacttctgc gagtcaaagg atacgatgtt 420
   tcatcagagg agctggctcc atacgctgat caagagggca tgaacttgca aacgattgat 480
   ctggcggcgg tcatcgagct gtacagagca gcacaggaga gagtggctga ggaagacagc 540
   actcttgaga aactgtatgt ctggaccagc acctttctga agcagcagtt gctggctggc 600
   gccattcctg accagaaatt gcacaaacag gtggagtact acttgaagaa ctaccacggc 660
   atattagata gaatgggagt tagaaaagga ctcgacctgt atgatgctgg ctattacaag 720
   gccctcaaag ctgcagatag gttggttgat ctatgcaatg aagaccttct agcatttgca 780
   aggcaagatt ttaatattaa ccaagcccaa caccgcaaag aacttgagca actgcaaagg 840
   tggtatgcag attgtaggtt ggacaaactc gagtttggaa gagatgtggt gcgtgtatcg 900
   aattttctga cttcagccat ccttggtgat ccagagcttt ctgaagtccg tctagtgttt 960
   gccaaacata ttgtgctagt gactaggata gatgattttt tcgatcatgg cgggcctaga 1020
   gaagaatcac acaagatcct tgaactaata aaagaatgga aagagaagcc agctggagaa 1080
   tatgtttcca aagaagttga gatcctatat accgcggtgt acaatacggt aaacgagttg 1140
   gcagagaggg caaatgttga acaagggcga aatgttgaac catttctacg tacactgtgg 1200
   gttcaaatac tgtcgatttt caagatagag ttggatacat ggagcgatga cacagcacta 1260
   accttggatg attacttgaa caactcatgg gtgtcgattg gttgtagaat ctgcattctc 1320
   atgtccatgc aattcattgg tatgaagtta ccagaagaaa tgcttctcag tgaagagtgc 1380
   gttgatttgt gtaggcatgt ttccatggtc gaccgtctgc tcaatgatgt ccaaactttt 1440
   gagaaggaac ggaaagaaaa tacaggaaac gctgtgagcc ttctgctagc agctcacaag 1500
   ggtgaaagag ccttcagtga agaggaagcc atagcaaaag cgaaatattt ggctgactgt 1560
   aacaggagaa gtctgatgca gattgtgtat aaaacaggaa ccattttccc aagaaaatgc 1620
   aaagatatgt tcttgaaggt gtgcaggatt ggttgctatt tgtatgcgag tggcgacgaa 1680
   tttacttccc ctcaacaaat gatggaagat atgaagtcat tagtttatga gcccctccaa 1740
   attcaccctc cacctgctaa ctaa 1764
<210> 9
   <211> 1704
   <212> DNA
   <213> Unknown
<220>
   <223> CfTPS3 from Coleus forskohlii
<220>
   <221> CfTPS3
   <222> (1)..(1704)
<400> 9
   atgatcacct ctaaatcatc tgcagctgtt aaatgcagcc tcaccacgcc aacagatttg        60
   atggggaaaa taaaagaggt cttcaacagg gaagtcgata cttctccggc agccatgact       120
   actcattcta cagatatacc ctctaatctc tgcataatcg acaccctcca gaggctggga       180
   atcgaccaat acttccaatc cgaaatcgac gctgttctac atgatacata caggttatgg       240
   caactgaaaa agaaagatat attttcggat attactactc atgcaatggc gttcagactt 300
   ttgcgagtca aaggatatga agttgcatca gacgaactgg ctccatacgc tgatcaagag 360
   cgcattaacc tgcaaaccat tgatgtgccg acagttgttg agctatacag agcagcacag 420
   gagagattaa ctgaagaaga tagcactctt gagaaactgt atgtttggac cagcgccttt 480
   ctgaagcagc agttgctcac tgatgccatt cctgacaaga aattgcacaa acaagtggaa 540
   tactacttga agaactacca tggcatatta gatagaatgg gagtgagacg aaacctcgac 600
   ctatatgaca taagccatta taaaagtctc aaagctgctc acaggttcta taatctgagt 660
   aatgaagata tcctagcatt tgcgaggcaa gattttaata ttagccaagc ccaacaccag 720
   aaagaacttc agcagctgca aaggtggtat gcagattgta ggttggacac gttgaaattt 780
   ggaagagatg tagtgcgtat aggaaatttt ctgacttcag caatgattgg tgatcctgaa 840
   ttgtctgacc tccgtctagc gtttgccaaa catatagtgc tcgtaacacg tattgatgat 900
   tttttcgatc acggtgggcc taaagaagaa tcatacgaga tccttgaatt agtaaaagaa 960
   tggaaagaga agccagcagg agaatatgtt tctgaagaag ttgaaatcct atttacagca 1020
   gtatacaata cagtaaacga gttggcagaa atggctcata tcgaacaagg acgaagcgtt 1080
   aaagaccttc tagttaaact gtgggttgaa atactatcag ttttcagaat agaattggat 1140
   acatggacca acgacacagc acttacctta gaagagtact tgtcacaatc ctgggtgtcc 1200
   attggctgca gaatctgcat tctcatatca atgcaattcc aaggtgtaaa attatctgat 1260
   gaaatgcttc agagtgaaga atgcactgat ttgtgtcggt atgtttcaat ggttgaccgg 1320
   ctgctcaacg atgtgcaaac ttttgagaag gaacgcaagg aaaatacagg aaatagtgtg 1380
   agccttctgc aagcagctca caaagatgaa agagtcatta atgaagagga agcttgtata 1440
   aaggtaaaag aattggctga atataacagg agaaaactga tgcagattgt ctacaaaaca 1500
   ggaaccattt tcccaagaaa atgcaaagat ctgtttttga aggcatgcag aattggttgt 1560
   tatttgtact caagtggcga cgaatttact tcgcctcaac aaatgatgga agatatgaag 1620
   tcactggttt atgaacccct accaatttct cctcctgaag ctaataatgc aagtggagaa 1680
   aaaatgagtt gtgtcagcaa ctag 1704
<210> 10
   <211> 2379
   <212> DNA
   <213> Euphorbia peplus
<220>
   <221> EpTPS8
   <222> (1)..(2379)
<400> 10
   atgcaagtct ctctctccct caccactggc tccgagcctt gcattaccag aatccatgct 60
   ccatctgatg caccattgaa acagcggaac aatgaaagag agaaggggac actagaacta      120
   aatgggaaag tttcgctgaa gaaaatggga gagatgctga ggacaataga aaatgtacct      180
   atagtaggta gtacgtcgtc gtatgataca gcatgggtcg gtatggtgcc ttgttcatcg      240
   aattcgtcga aaccgctatt tccagaaagc ttgaaatgga taatggagaa tcaaaatcca      300
   gaagggaatt gggcagttga tcatgctcat catcctcttc ttcttaaaga ctctctttct      360
   tccactcttg cttgtgtcct tgctttgcac aaatggaatt tggctcccca gcttgttcac      420
   tccggtttgg acttcatcgg ctctaatcta tgggcagcta tggactttcg acaacgatct      480
   cctctcggat ttgatgttat atttccagga atgatccacc aagctattga tttgggcatc      540
   aatcttcctt tcaacaactc ttcaattgag aacatgctca ctaacccact tttggacatt      600
   caaagttttg aagcaggaaa aactagccat attgcatact ttgccgaggg attaggaagt      660
   agattaaagg attgggaaca acttcttcaa tatcaaacaa gtaacggttc gctcttcaat      720
   tcaccttcta caactgctgc cgctgctatt catctacgtg acgaaaaatg tcttaattac      780
   ttgcattctc taaccaaaca attcgataat ggtgctgttc caacacttta tcctctcgat      840
   gcgcgtacca gaatctccat aatcgatagt ttggaaaagt ttgggatcca ttcacatttc      900
   atccaagaaa tgacaattct actagatcaa atatacagct tctggaaaga agggaatgaa      960
   gaaatattta aagaccctgg atgttgtgca acagcattcc gactgctgcg aaagcatggt     1020
   tatgatgttt cttcagattc cttggcagag tttgagaaaa aagagatatt ttaccattca     1080
   tcagcagcta gtgcacacga aatcgatacc aagtctattc tagaattatt cagagcttcc     1140
   caaatgaaaa ttttgcaaaa tgaaccaata ctcgacagaa tttacgattg gactagcatt     1200
   tttctgagag accagctagt gaaaggtcta atcgaaaaca agagtctgta cgaagaagtt     1260
   aattttgctt tgggacatcc atttgctaat ctggatagac tcgaagctcg ttcttacatc     1320
   gacaattacg atccatatga tgtcccactt cttaagacat cttacaggtc atccaatatt     1380
   gataacaaag atctatggac aattgcattc caagatttca acaagtgcca agccttgcac     1440
   cgtgtggaac ttgattatct ggagaaatgg gtgaaagaat acaaattgga cactctgaag     1500
   tgggcaaggc agaagactga gtatgcatta tttacgatag gcgcaatcct ttcggagcct     1560
   gaatacgctg atgctcgcat ctcatggtca cagaatactg tttttgtgac tattgttgat     1620
   gatttctttg actatggtgg ttcgttggat gaatgtcgta acctcattaa ccttatgcac     1680
   aagtgggatg atcacttaac cgttggattc ttgtcggaaa aagtggaaat cgtattttat     1740
   tcgatgtatg gcacactcaa tgaccttgct gccaaagccg aagtacgaca aggccgatgt     1800
   gttcgaagtc acttagttaa tttatggatc tgggtgatgg aaaacatgtt aaaggagaga     1860
   gaatgggcag attacaatct ggtgcctaca ttttacgagt acgtagccgc tggacatata     1920
   actatcggct taggacctgt gcttcttatt gccctctatt ttatggggta tccgctttct     1980
   gaggatgtgg ttcaaagtca agaatacaag ggtgtttatt tgaatgtcag catcattgct     2040
   cgacttctaa atgatcgcgt aactgttaag agggaaagtg cgcaaggaaa gcttaatggt     2100
   gtgtcattgt tcgtcgaaca tggtcgtggc gcggttgatg aggaaactag tatgaaggaa     2160
   gtagaaagac tggtagagag ccataagaga gaattattaa gattgattgt gcagaaaacg     2220
   gaaggcagtg tcgtcccgca aagttgcaaa gatctagctt ggagggttag caaagttttg     2280
   caccttctat atatggatga tgatggtttt acatgtcctg tgaagatgct taatgctaca     2340
   aatgcaattg tcaacgaacc actcctttta acttcataa                            2379
<210> 11
   <211> 4
   <212> PRT
   <213> Unknown
<220>
   <223> Class II diterpene synthase domain
<220>
   <221> DOMAIN
   <222> (1)..(4)
   <223> X: any amino acid
<400> 11
   Asp Xaa Asp Asp
   1
<210> 12
   <211> 5
   <212> PRT
   <213> Unknown
<220>
   <223> Class I diterpene synthase domain
<220>
   <221> DOMAIN
   <222> (1)..(5)
   <223> X: any amino acid
<400> 12
   Asp Asp Xaa Xaa Asp
   1               5
<210> 13
   <211> 28
   <212> DNA
   <213> Artificial sequence
<220>
   <223> Bri046 forward primer for amplification of CfTPS1
<220>
   <221> primer_bind
   <222> (1)..(28)
   <223> CfTPS1_F
<400> 13
   cagaatgggg tctctatcca ctatgaac 28
<210> 14
   <211> 22
   <212> DNA
   <213> Artificial sequence
<220>
   <223> Bri047 reverse primer for amplification of CfTPS1
<220>
   <221> primer_bind
   <222> (1)..(22)
   <223> CfTPS1_R
<400> 14
   cagcatattc aggcgactgg tt 22
<210> 15
   <211> 30
   <212> DNA
   <213> Artificial sequence
<220>
   <223> Bri048 forward primer for amplification of CfTPS2
<220>
   <221> primer_bind
   <222> (1)..(30)
   <223> CfTPS2_F
<400> 15
   agattgagga ttccattgag tacgtgaagg 30
<210> 16
   <211> 30
   <212> DNA
   <213> Artificial sequence
<220>
   <223> Bri049 reverse primer for amplification of CfTPS2
<220>
   <221> primer_bind
   <222> (1)..(30)
   <223> CfTPS2_R
<400> 16
   gaagtttaat atccttcatt ctttattaca 30
<210> 17
   <211> 27
   <212> DNA
   <213> Artificial sequence
<220>
   <223> Bri099 forward primer for amplification of CfTPS3
<220>
   <221> primer_bind
   <222> (1)..(27)
   <223> CfTPS3_F
<400> 17
   agctccattc aactagagtc atgtcgt 27
<210> 18
   <211> 28
   <212> DNA
   <213> Artificial sequence
<220>
   <223> Bri100 reverse primer for amplification of CfTPS3
<220>
   <221> primer_bind
   <222> (1)..(28)
   <223> CfTPS3_R
<400> 18
   ttcatctggc ttaactagtt gctgacac 28
<210> 19
   <211> 25
   <212> DNA
   <213> Artificial sequence
<220>
   <223> Bri0101 forward primer for amplification of CfTPS4
<220>
   <221> primer_bind
   <222> (1)..(25)
   <223> CfTPS4_F
<400> 19
   gtgcactctc caccaacgat aaact 25
<210> 20
   <211> 25
   <212> DNA
   <213> Artificial sequence
<220>
   <223> Bri102 reverse primer for amplification of CfTPS4
<220>
   <221> primer_bind
   <222> (1)..(25)
   <223> CfTPS4_R
<400> 20
   gcttcacagc ctatgaatac atgat 25
<210> 21
   <211> 22
   <212> DNA
   <213> Artificial sequence
<220>
   <223> Bri051 forward primer for amplification of CfTPS14
<220>
   <221> primer_bind
   <222> (1)..(22)
   <223> CfTPS14_F
<400> 21
   tatgacacgg catgggttgc ta 22
<210> 22
   <211> 29
   <212> DNA
   <213> Artificial sequence
<220>
   <223> Bri052 reverse primer for amplification of CfTPS14
<220>
   <221> primer_bind
   <222> (1)..(29)
   <223> CfTPS14_R
<400> 22
   tcactcaaaa tttattctaa gacaagagg 29
<210> 23
   <211> 26
   <212> DNA
   <213> Artificial sequence
<220>
   <223> Bri064 forward primer for E. coli expression of CfTPS1
<220>
   <221> primer_bind
   <222> (1)..(26)
   <223> CfTPS1_PciI_pmF
<400> 23
   gctttagcaa catgtcatgg atgaac 26
<210> 24
   <211> 22
   <212> DNA
   <213> Artificial sequence
<220>
   <223> Bri065 reverse primer for E. coli expression of CfTPS1
<220>
   <221> primer_bind
   <222> (1)..(22)
   <223> CfTPS1_XhoI_R
<400> 24
   cagcactcga gggcgactgg tt 22
<210> 25
   <211> 25
   <212> DNA
   <213> Artificial sequence
<220>
   <223> Bri068 forward primer for E. coli expression of CfTPS2
<220>
   <221> primer_bind
   <222> (1)..(25)
   <223> CfTPS2_BspHI_pmF
<400> 25
   cacaagtaat catgagtcga gttgc 25
<210> 26
   <211> 23
   <212> DNA
   <213> Artificial sequence
<220>
   <223> Bri069 reverse primer for E. coli expression of CfTPS2
<220>
   <221> primer_bind
   <222> (1)..(23)
   <223> CfTPS2_XhoI_R
<400> 26
   ccaatgttct cgagcactgg ttc 23
<210> 27
   <211> 37
   <212> DNA
   <213> Artificial sequence
<220>
   <223> Bri146 forward primer for E. coli expression of CfTPS3
<220>
   <221> primer_bind
   <222> (1)..(37)
   <223> CfTPS3_NcoI_inFusion_F
<400> 27
   aggagatata ccatggctcc gatgatcacc tctaaat 37
<210> 28
   <211> 35
   <212> DNA
   <213> Artificial sequence
<220>
   <223> Bri147 reverse primer for E. coli expression of CfTPS3
<220>
   <221> primer_bind
   <222> (1)..(35)
   <223> CfTPS3_XhoI_inFusion_R
<400> 28
   ggtggtggtg ctcgaggttg ctgacacaac tcatt 35
<210> 29
   <211> 26
   <212> DNA
   <213> Artificial sequence
<220>
   <223> Bri117 forward primer for E. coli expression of CfTPS4
<220>
   <221> primer_bind
   <222> (1)..(26)
   <223> CfTPS4_BspHI_pmF
<400> 29
   catcctttgt catgaaatgc agcctc 26
<210> 30
   <211> 29
   <212> DNA
   <213> Artificial sequence
<220>
   <223> Bri118 reverse primer for E. coli expression of CfTPS4
<220>
   <221> primer_bind
   <222> (1)..(29)
   <223> CfTPS4_NotI_R
<400> 30
   ttgttaggcg gccgctggag ggtgaattt 29
<210> 31
   <211> 25
   <212> DNA
   <213> Artificial sequence
<220>
   <223> Bri066 forward primer for E. coli expression of CfTPS14
<220>
   <221> primer_bind
   <222> (1)..(25)
   <223> CfTPS14_NcoI_pmF
<400> 31
   ttacgccatg gcttccctgg aagtt 25
<210> 32
   <211> 27
   <212> DNA
   <213> Artificial sequence
<220>
   <223> Bri 067 reverse primer for E. coli expression of CfTPS14
<220>
   <221> primer_bind
   <222> (1)..(27)
   <223> CfTPS14_XhoI_R
<400> 32
   actcaactcg agttctaaga caagagg 27
<210> 33
   <211> 33
   <212> DNA
   <213> Artificial sequence
<220>
   <223> oSSB156 forward primer for tobacco expression of CfTPS1
<220>
   <221> primer_bind
   <222> (1)..(33)
   <223> CfTPS1 (f)
<400> 33
   ggcttaauat ggggtctcta tccactatga acc 33
<210> 34
   <211> 26
   <212> DNA
   <213> Artificial sequence
<220>
   <223> oSSB157 reverse primer for tobacco expression of CfTPS1
<220>
   <221> primer_bind
   <222> (1)..(26)
   <223> CfTPS1 (rc)
<400> 34
   ggtttaautc aggcgactgg ttcgaa 26
<210> 35
   <211> 34
   <212> DNA
   <213> Artificial sequence
<220>
   <223> oSSB158 forward primer for tobacco expression of CfTPS2
<220>
   <221> primer_bind
   <222> (1)..(34)
   <223> CfTPS2 (f)
<400> 35
   ggcttaauat gaaaatgttg atgatcaaaa gtca 34
<210> 36
   <211> 35
   <212> DNA
   <213> Artificial sequence
<220>
   <223> oSSB159 reverse primer for tobacco expression of CfTPS2
<220>
   <221> primer_bind
   <222> (1)..(35)
   <223> CfTPS2 (rc)
<400> 36
   ggtttaautc agaccactgg ttcaaatagt acttg 35
<210> 37
   <211> 24
   <212> DNA
   <213> Artificial sequence
<220>
   <223> oSSB178 forward primer for tobacco expression of CfTPS3
<220>
   <221> primer_bind
   <222> (1)..(24)
   <223> CfTPS3 (f)
<400> 37
   ggcttaauat gtcgtccctc gccg 24
<210> 38
   <211> 35
   <212> DNA
   <213> Artificial sequence
<220>
   <223> oSSB179 reverse primer for tobacco expression of CfTPS3
<220>
   <221> primer_bind
   <222> (1)..(35)
   <223> CfTPS3 (rc)
<400> 38
   ggtttaauct agttgctgac acaactcatt ttttc 35
<210> 39
   <211> 31
   <212> DNA
   <213> Artificial sequence
<220>
   <223> oSSB180 forward primer for tobacco expression of CfTPS4
<220>
   <221> primer_bind
   <222> (1)..(31)
   <223> CfTPS4 (f)
<400> 39
   ggcttaauat gtcaatcacc atcaaccttc g 31
<210> 40
   <211> 32
   <212> DNA
   <213> Artificial sequence
<220>
   <223> oSSB181 reverse primer for tobacco expression of CfTPS4
<220>
   <221> primer_bind
   <222> (1)..(32)
   <223> CfTPS4 (rc)
<400> 40
   ggtttaautt agttagcagg tggagggtga at 32
<210> 41
   <211> 31
   <212> DNA
   <213> Artificial sequence
<220>
   <223> oSSB160 forward primer for tobacco expression of CfTPS14
<220>
   <221> primer_bind
   <222> (1)..(31)
   <223> CfTPS14 (f)
<400> 41
   ggcttaauat gtctctcccg ctctctactt g 31
<210> 42
   <211> 41
   <212> DNA
   <213> Artificial sequence
<220>
   <223> oSSB161 reverse primer for tobacco expression of CfTPS14
<220>
   <221> primer_bind
   <222> (1)..(41)
   <223> CfTPS14 (rc)
<400> 42
   ggtttaautt attctaagac aagaggttga taaataattg c 41
<210> 43
   <211> 22
   <212> DNA
   <213> Artificial sequence
<220>
   <223> CfTPS1 forward primer for qPCR
<220>
   <221> primer_bind
   <222> (1)..(22)
   <223> CfTPS1.for
<400> 43
   tagtctggaa gggctggaga at 22
<210> 44
   <211> 24
   <212> DNA
   <213> Artificial sequence
<220>
   <223> CfTPS1 reverse primer for qPCR
<220>
   <221> primer_bind
   <222> (1)..(24)
   <223> CfTPS1.rev
<400> 44
   ttggtagcat ttaggatcac gagt 24
<210> 45
   <211> 28
   <212> DNA
   <213> Artificial sequence
<220>
   <223> CfTPS2 forward primer for qPCR
<220>
   <221> primer_bind
   <222> (1)..(28)
   <223> CfTPS2.for
<400> 45
   gacatattat cgcactatga atacaccg 28
<210> 46
   <211> 29
   <212> DNA
   <213> Artificial sequence
<220>
   <223> CfTPS2 reverse primer for qPCR
<220>
   <221> primer_bind
   <222> (1)..(29)
   <223> CfTPS2.rev
<400> 46
   ctaacttcac caatgtttgt atttcgagc 29
<210> 47
   <211> 29
   <212> DNA
   <213> Artificial sequence
<220>
   <223> CfTPS3 forward primer for qPCR
<220>
   <221> primer_bind
   <222> (1)..(29)
   <223> CfTPS3.for
<400> 47
   catgcagaat tggttgttat ttgtactca 29
<210> 48
   <211> 27
   <212> DNA
   <213> Artificial sequence
<220>
   <223> CfTPS3 reverse primer for qPCR
<220>
   <221> primer_bind
   <222> (1)..(27)
   <223> CfTPS3.rev
<400> 48
   tttctccact tgcattatta gcttcag 27
<210> 49
   <211> 25
   <212> DNA
   <213> Artificial sequence
<220>
   <223> CfTPS4 forward primer for qPCR
<220>
   <221> primer_bind
   <222> (1)..(25)
   <223> CfTPS4.for
<400> 49
   aacggaaaga aaatacagga aacgc 25
<210> 50
   <211> 26
   <212> DNA
   <213> Artificial sequence
<220>
   <223> CfTPS4 reverse primer for qPCR
<220>
   <221> primer_bind
   <222> (1)..(26)
   <223> CfTPS4.rev
<400> 50
   cagccaaata tttcgctttt gctatg 26
<210> 51
   <211> 27
   <212> DNA
   <213> Artificial sequence
<220>
   <223> CfTPS14 forward primer for qPCR
<220>
   <221> primer_bind
   <222> (1)..(27)
   <223> CfTPS14 (f)
<400> 51
   atgcatatgt atcatttgct ctagggc 27
<210> 52
   <211> 30
   <212> DNA
   <213> Artificial sequence
<220>
   <223> CfTPS14 reverse primer for qPCR
<220>
   <221> primer_bind
   <222> (1)..(30)
   <223> CfTPS14 .rev
<400> 52
   ggatttgaat agattgtggt agtcagcatg 30
<210> 53
   <211> 20
   <212> DNA
   <213> Artificial sequence
<220>
   <223> CfEF1a forward primer for qPCR reference
<220>
   <221> primer_bind
   <222> (1)..(20)
   <223> CfEF1a (f)
<400> 53
   tgcatcacga ggctctccag 20
<210> 54
   <211> 20
   <212> DNA
   <213> Artificial sequence
<220>
   <223> CfEF1a reverse primer for qPCR reference
<220>
   <221> primer_bind
   <222> (1)..(20)
   <223> CfEF1a (rc)
<400> 54
   ggcaacaaac ccacgcttca 20
<210> 55
   <211> 23
   <212> DNA
   <213> Artificial sequence
<220>
   <223> CfH3 forward primer for qPCR reference
<220>
   <221> primer_bind
   <222> (1)..(23)
   <223> CfH3 (f)
<400> 55
   gagattcgca agtaccagaa gag 23
<210> 56
   <211> 23
   <212> DNA
   <213> Artificial sequence
<220>
   <223> CfH3 reverse primer for qPCR reference
<220>
   <221> primer_bind
   <222> (1)..(23)
   <223> CfH3 (rc)
<400> 56
   aatcgcagat cagtcttgaa gtc 23
<210> 57
   <211> 21
   <212> DNA
   <213> Artificial sequence
<220>
   <223> CfTIF4a forward primer for qPCR reference
<220>
   <221> primer_bind
   <222> (1)..(21)
   <223> CfTIF4a (f)
<400> 57
   ctatgatctg ccaactcagc c 21
<210> 58
   <211> 21
   <212> DNA
   <213> Artificial sequence
<220>
   <223> CfTIF4a reverse primer for qPCR reference
<220>
   <221> primer_bind
   <222> (1)..(21)
   <223> CfTIF4a (rc)
<400> 58
   ccttggtcac gaagtttatg g 21
<210> 59
   <211> 23
   <212> DNA
   <213> Artificial sequence
<220>
   <223> gCfTIF4a forward primer for qPCR gDNA check
<220>
   <221> primer_bind
   <222> (1)..(23)
   <223> gCfTIF4a (f)
<400> 59
   tttgacatgy tgagaaggca gtc 23
<210> 60
   <211> 21
   <212> DNA
   <213> Artificial sequence
<220>
   <223> gCfTIF4a reverse primer for qPCR gDNA check
<220>
   <221> primer_bind
   <222> (1)..(21)
   <223> gCfTIF4a (rc)
<400> 60
   aacatagaac tgcttgatac c 21
<210> 61
   <211> 23
   <212> DNA
   <213> Artificial sequence
<220>
   <223> gCfEFla forward primer for qPCR gDNA check
<220>
   <221> primer_bind
   <222> (1)..(23)
   <223> gCfEFla (f)
<400> 61
   tactactgca ctgtcattga tgc 23
<210> 62
   <211> 22
   <212> DNA
   <213> Artificial sequence
<220>
   <223> gCfEFla reverse primer for qPCR gDNA check
<220>
   <221> primer_bind
   <222> (1)..(22)
   <223> gCfEFla (rc)
<400> 62
   tggacctctc aatcatgttg tc 22
<210> 63
   <211> 1710
   <212> DNA
   <213> Unknown
<220>
   <223> CfTPS3 codon optimised for E. coli
<220>
   <221> CfTPS3_co
   <222> (1)..(1710)
   <223> CfTPS3 from Coleus forskohlii, gene optimised
<400> 63
   atgggcatta ccagcaagtc ctccgcagca gtcaaatgta gcctgaccac gccgaccgac      60
   ctgatgggca agattaaaga agttttcaat cgtgaagtgg ataccagccc ggcagcaatg     120
   accacgcaca gtacggacat cccgtccaac ctgtgcatta tcgataccct gcaacgtctg     180
   ggcatcgacc aatattttca gtctgaaatt gatgcagttc tgcatgacac ctaccgcctg     240
   tggcagctga aaaagaaaga tatctttagt gacatcacca cgcacgcgat ggccttccgt     300
   ctgctgcgtg tcaaaggtta tgaagtggcg agcgatgaac tggcaccgta cgctgaccaa     360
   gaacgtatca atctgcagac cattgatgtc ccgacggtgg ttgaactgta tcgtgcagct     420
   caggaacgcc tgaccgaaga agatagtacg ctggaaaagc tgtacgtttg gaccagcgcg     480
   tttctgaaac agcaactgct gacggatgcc attccggaca agaaactgca taagcaggtt     540
   gaatattacc tgaaaaacta tcacggcatc ctggatcgca tgggtgtccg tcgcaatctg     600
   gatctgtatg acattagcca ttacaagtct ctgaaagcgg cccaccgttt ctacaacctg     660
   tcaaatgaag atatcctggc atttgctcgc caagacttca acatttcgca ggcacaacat     720
   cagaaagaac tgcagcaact gcagcgttgg tatgctgatt gtcgcctgga caccctgaaa     780
   tttggccgtg atgtcgtgcg catcggtaat ttcctgacga gcgccatgat tggcgatccg     840
   gaactgtctg acctgcgtct ggcgtttgcc aaacatatcg ttctggtcac ccgcattgat     900
   gactttttcg atcacggcgg tccgaaggaa gaaagctatg aaatcctgga actggtgaaa     960
   gaatggaagg aaaaaccggc gggcgaatat gtctctgaag aagtggaaat cctgtttacc    1020
   gccgtttaca acacggtcaa tgaactggcg gaaatggccc acattgaaca gggtcgtagt    1080
   gtgaaggatc tgctggtgaa actgtgggtt gaaatcctgt ccgttttccg cattgaactg    1140
   gatacctgga cgaacgacac cgcactgacg ctggaagaat atctgagtca gtcctgggtg    1200
   tcaattggct gccgcatttg tatcctgatt tcgatgcaat ttcagggtgt taaactgtca    1260
   gatgaaatgc tgcagtcgga agaatgcacc gacctgtgtc gttacgtgag catggttgat    1320
   cgcctgctga atgacgtcca aaccttcgaa aaggaacgta aagaaaacac gggcaattca    1380
   gtgtcgctgc tgcaggcagc tcataaagat gaacgcgtta tcaacgaaga agaagcgtgc    1440
   attaaggtca aagaactggc tgaatataat cgtcgcaagc tgatgcagat cgtgtacaaa    1500
   accggtacga tttttccgcg taagtgtaaa gacctgtttc tgaaagcgtg ccgcattggc    1560
   tgttatctgt acagctctgg tgatgaattt accagcccgc agcaaatgat ggaagacatg    1620
   aaatctctgg tttatgaacc gctgccgatt agcccgccgg aagccaacaa tgcgtcaggt    1680
   gaaaagatgt cgtgtgtctc aaacctcgag                                     1710